(19) Europäisches Patentamt
European Patent Office
Office européen des brevets

(11) Veröffentlichungsnummer: **0 064 026**
**B1**

# (12) EUROPÄISCHE PATENTSCHRIFT

(45) Veröffentlichungstag der Patentschrift: **03.07.85**

(51) Int. Cl.⁴: **A 61 K 31/36,** C 07 D 317/70, C 07 D 409/06

(21) Anmeldenummer: **82810162.6**

(22) Anmeldetag: **19.04.82**

(54) **Neue dioxaheterocyclische Verbindungen, Verfahren zu deren Herstellung und diese enthaltende pharmazeutische Zusammensetzungen.**

(30) Priorität: **24.04.81 CH 2697/81**

(43) Veröffentlichungstag der Anmeldung: **03.11.82 Patentblatt 82/44**

(45) Bekanntmachung des Hinweises auf die Patenterteilung: **03.07.85 Patentblatt 85/27**

(84) Benannte Vertragsstaaten: **AT BE CH DE FR GB IT LI LU NL SE**

(56) Entgegenhaltungen: **DE - A - 2 426 505**

(73) Patentinhaber: **CIBA-GEIGY AG, Postfach, CH-4002 Basel (CH)**

(72) Erfinder: **Habicht, Ernst, Dr., Bienenstrasse 13, CH-4104 Oberwil (CH)**
Erfinder: **Zbinden, Paul, Im Rainacker 6, CH-4108 Witterswil (CH)**

**EP 0 064 026 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue dioxaheterocyclische Verbindungen, Verfahren zu deren Herstellung und diese enthaltende pharmazeutische Zusammensetzungen, sowie die Verwendung dieser neuen Stoffe und pharmazeutischen Zusammensetzungen.

In der Offenlegungsschrift Nr. 2 426 505 werden 1,3-Benzodioxol-2-carbonsäuren, deren Salze, Ester und Amide, die durch einen gegebenenfalls substituierten Phenylrest am aromatischen Teil des 1,3-Benzodioxolkerns substituiert sind, beschrieben, die sich zur Herabsetzung von Cholesterin und verschiedene Triglyceride enthaltenden Blutlipoproteinen eignen, wogegen die erfindungsgemässen substituierten Oxo-indano-2-carbonsäuren und deren Derivate diuretische, saliuretische und uricosurische Wirkungen aufweisen.

Die erfindungsgemässen neuen Verbindungen entsprechen der allgemeinen Formel I

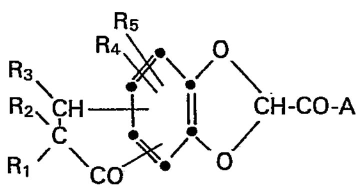

(I)

in denen $R_1$ durch Niederalkyl, Niederalkoxy oder Halogen substituiertes Phenyl oder Pyridyl, $R_2$ primäres Niederalkyl, $R_3$ und $R_4$ unabhängig voneinander Wasserstoff oder Niederalkyl, $R_5$ Wasserstoff bedeutet und A den Rest -O-$R_6$, worin $R_6$ für Wasserstoff oder einen unsubstituierten oder durch Niederalkoxy oder Halogen substituierten Alkylrest mit höchstens 12 C-Atomen, 2- oder 3-Niederalkenyl, 2-Niederalkinyl, Phenylniederalkyl oder Cinnamyl, in welchen der Phenylrest in gleicher Weise wie unter $R_1$ substituiert sein kann, steht, oder den Rest $-N\genfrac{}{}{0pt}{}{R_7}{R_8}$, worin $R_7$ und $R_8$ unabhängig voneinander für Wasserstoff oder Niederalkyl, oder unter sich verbunden und zusammen mit dem anliegenden Stickstoffatom für gegebenenfalls niederalkylsubstituiertes Tetra-bis Hexamethylenimino oder 4-Morpholinyl stehen, bedeutet, und der angegebene zweiwertige Rest entweder in 4,5- oder in 5,6-Stellung und entsprechend $R_4$ und $R_5$ in den Stellungen 6 und 7 bzw. 4 und 7 des Benzodioxol-Molekülteils stehen, als Racematgemische, Racemate, diastereomere Antipodengemische oder optische Antipoden, sowie Salze von Verbindungen der allgemeinen Formel I, in denen A = $OR_6$ und darin $R_6$ Wasserstoff bedeutet, mit Basen, wobei die mit «nieder» bezeichneten Reste höchstens 7 Kohlenstoffatome aufweisen.

Der Rest $R_1$ als Pyridyl kann beispielsweise ein 2-, 3- oder auch 4-Pyridylrest sein.

Als substituierter Phenyl- oder heteroaromatischer Pyridylrest ist $R_1$ z.B. durch Halogen, wie Fluor, Brom, Jod oder insbesondere Chlor, durch Niederalkyl, wie z.B. Äthyl, Propyl, Isopropyl, Butyl, Isobutyl, tert.-Butyl oder insbesondere Methyl, und/oder Niederalkoxy, wie Äthoxy, Propoxy, Isopropoxy, Butoxy, Isobutoxy und insbesondere Methoxy, und/oder Trifluormethyl ein- oder mehrfach, vorzugsweise höchstens dreifach substituiert.

Als Niederalkyl sind $R_2$ und $R_3$ z.B. Äthyl, Propyl, Butyl oder Isobutyl und insbesondere Methyl; vor allem $R_3$ kann aber z.B. auch Isopropyl oder tert.-Butyl sein.

$R_4$ ist als Niederalkyl z.B. einer der vorgenannten Reste, insbesondere Methyl.

$R_6$ als Alkyl ist z.B. Propyl, Isopropyl, Butyl, Isobutyl, Pentyl, Isopentyl, Neopentyl, Hexyl, Heptyl, Octyl, Nonyl, Decyl, Dodecyl und vor allem Methyl oder Äthyl; Niederalkenyl $R_6$ z.B. Allyl, 1- oder 2-Methylallyl, 2-Butenyl oder 3-Butenyl; Niederalkinyl z.B. 2-Propinyl; Niederalkoxyniederalkyl insbesondere 2- oder 3-Niederalkoxyniederalkyl, wie z.B. 2-Methoxy-, 2-Äthoxy-, 2-Isopropoxy- oder 2-Butoxyäthyl, 2- oder 3-Methoxypropyl, 2- oder 3-Äthoxypropyl, ferner 3- oder 4-Methoxybutyl oder 3- oder 4-Äthoxybutyl, und halogeniertes Niederalkyl insbesondere geminal polyhalogeniertes Niederalkyl wie 2,2,2-Trifluor- oder 2,2,2-Trichloräthyl. Phenylniederalkyl $R_6$ ist z.B. Benzyl, 2-Phenyläthyl, 2- oder 3-Phenylpropyl oder 2-, 3- oder 4-Phenylbutyl.

Salze der neuen Verbindungen sind insbesondere Salze von Verbindungen der allgemeinen Formel I, worin A = $OR_6$ und darin $R_6$ Wasserstoff bedeutet, mit Basen, vor allem pharmazeutisch verwendbare Salze solcher Verbindungen mit Basen. Als solche Salze mit Basen kommen beispielsweise Alkalimetall- oder Erdalkalimetallsalze, wie Natrium-, Kalium-, Calcium- oder Magnesiumsalze, ferner Ammoniumsalze mit Ammoniak oder organischen Aminen, wie Mono- oder Diniederalkylaminen, z.B. Methylamin, Äthylamin, Dimethylamin oder Diäthylamin, oder Mono-, Di- oder Tri(hydroxyalkyl)-aminen, z.B. 2-Aminoäthanol, 2-(Dimethylamino)-äthanol, 2-(Diäthylamino)-äthanol, 2,2'-Iminodiäthanol oder 2,2',2''-Nitrilotri-äthanol in Betracht.

Als Säureadditionssalze, insbesondere pharmazeutisch annehmbare Säureadditionssalze, von Verbindungen der allgemeinen Formel I, in denen $R_1$ basischen Charakter aufweist, kommen beispielsweise solche mit geeigneten anorganischen Säuren, wie Halogenwasserstoffsäuren, z.B. Chlorwasserstoffsäure oder Bromwasserstoffsäure, ferner Salpetersäure, Schwefelsäure oder Phosphorsäure, oder mit geeigneten organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, oder organischen Sulfonsäuren, wie gegebenenfalls Hydroxy enthaltenden Niederalkansulfonsäuren, z.B. Methansulfonsäure, Äthansulfonsäure, 2-Hydroxyäthansulfonsäure oder Äthan-1,2-disulfonsäure, oder Arylsulfonsäuren, z.B. Benzolsulfonsäure, 4-Methyl-benzolsulfonsäure oder Naphthalin-2-sulfonsäure, oder weiteren sauren Substanzen, wie Ascorbinsäure, in Frage.

Die neuen dioxaheterocyclischen Verbindungen der allgemeinen Formel I und ihre Salze besitzen wertvolle pharmakologische Eigenschaften. Sie wirken insbesondere diuretisch und natriuretisch an der Ratte im Dosisbereich von 10 bis 1000 mg/kg per os und am Hund im Dosisbereich von 1 bis 20 mg/kg per os, wie durch Sammeln des Harns während 3 Stunden nach Verabreichung (Ratte) bzw. stündlich während 5 Stunden nach Verabreichung (Hund) und Bestimmung des Harnvolumens und der Natrium-, Kalium- und Chlorionen festgestellt werden kann. Hierbei wird die Kalium-Ausscheidung erst in höheren Dosen und weniger stark gesteigert als die Natrium-Ausscheidung; hervorzuheben ist auch die gute Verträglichkeit der Verbindungen der allgemeinen Formel I. Beispielsweise wird durch die Verabreichung von 10 mg/kg per os Natriumsalz der selber bei 161-163° schmelzenden 7,8-Dihydro-5,7-dimethyl-6-oxo-7-phenyl-6H-indeno[4,5-d]-1,3-dioxol-2-carbonsäure und von 10 mg/kg per os Natriumsalz der 7,8-Dihydro-5,7-dimethyl-7-(2-fluorphenyl)-6-oxo-6H-indeno[4,5-d]-1,3-dioxol-2-carbonsäure an Ratten (6 bzw. 3 Tiere pro Dosis) im Vergleich zu unbehandelten Kontrolltieren die Ausscheidung von Natriumionen auf das 5fache bzw. 6fache, von Kaliumionen auf das 3fache bzw. 1,8fache und von Chlor-ionen auf das 8fache bzw. 8,5fache gesteigert. Am Hund wird durch die Verabreichung von 1 bzw. 5 mg/kg per os des ersten vorgenannten Natriumsalzes (3 Tiere pro Dosis) die durchschnittliche Ausscheidung pro Minute während der ersten 5 Stunden nach Verabreichung im Vergleich zur durchschnittlichen Ausscheidung pro Minute während der letzten Stunde von Verabreichung bezüglich der Natriumionen auf das 17fache bzw. 39fache, bezüglich der Kaliumionen auf das 1,5fache bzw. 2,5fache, bezüglich der Chlorionen auf das 12fache bzw. 29fache und bezüglich des Harnvolumens auf das 2fache bzw. 8fache gesteigert. Weiter besitzen Verbindungen der allgemeinen Formel I, wie z.B. das erste der vorgenannten Natriumsalze oder das Natriumsalz der 6,7-Dihydro-6-methyl-5-oxo-6-phenyl-5H-indeno[5,6-d]-1,3-dioxol-2-carbonsäure, uricosurische Wirksamkeit, wie sich z.B. anhand von Versuchen am Cebus-Affen (Cebus apella) zeigen lässt. Bei diesen Versuchen wird den Versuchstieren in Pentobarbital-Narkose Polyfructosan in Ringer-Lösung intravenös infundiert und die Prüfsubstanz als wässrige Lösung in ansteigenden Dosen intravenös injiziert. Vor der ersten Prüfsubstanz-Verabreichung und nach jeder Prüfsubstanzdosis wird während je 2 bis 4 10minütigen Perioden der Harn gesammelt und vor der ersten und nach der letzten Sammelperiode jeweils arteriell Blut entnommen. Die Harnsäure- und Polyfructosan-clearance wird aus deren Plasma- und Urin-Konzentration berechnet und schliesslich die fraktionelle Harnsäureausscheidung (Fractional excretion of uric acid, $FE_{UE}$) als Quotient von Harnsäure-clearance und glomerulärer Filtrationsrate bestimmt. Verbindungen der allgemeinen Formel I zeigen in diesem Test Wirkungen im Dosisbereich von 2 bis 10 mg/kg i.v., z.B. bewirkt die Verabreichung von 10 mg/kg des ersten oder dritten der vorgenannten Natriumsalze eine Verdoppelung der fraktionellen Harnsäureausscheidung. Entsprechend können die Verbindungen der allgemeinen Formel I und ihre pharmazeutisch annehmbaren Salze als ka-liumneutrale Diuretika mit urikosurischer Zusatzwirkung, z.B. zur Behandlung von Oedemen und der Hypertension verwendet werden.

Die Erfindung betrifft ganz besonders Verbindungen der allgemeinen Formel I, in denen $R_1$ durch Niederalkyl, Niederalkoxy oder Halogen substituiertes oder unsubstituiertes Phenyl oder Pyridyl, $R_2$ primäres Niederalkyl, $R_3$ Wasserstoff oder Niederalkyl, $R_5$ Wasserstoff und A $OR_6$ und darin $R_6$ Wasserstoff oder Niederalkyl bedeutet, und $R_4$ Niederalkyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, diastereomere Antipodengemische oder insbesondere als Racemate oder optische Antipoden, sowie die pharmazeutisch annehmbaren Salze von solchen Verbindungen, in denen $A = OR_6$ und darin $R_6$ Wasserstoff ist, mit Basen, wobei die mit «nieder» bezeichneten Reste höchstens 7 Kohlenstoffatome aufweisen.

Die Erfindung betrifft vor allem Verbindungen der allgemeinen Formel I, in denen $R_1$ durch Niederalkyl, Niederalkoxy oder Halogen substituiertes oder unsubstituiertes Phenyl, $R_2$ primäres Niederalkyl, $R_3$ und $R_5$ je Wasserstoff und A $OR_6$ und darin $R_6$ Wasserstoff oder Niederalkyl bedeutet und $R_4$ Niederalkyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, diastereomere Antipodengemische oder insbesondere als Racemate oder optische Antipoden, sowie die pharmazeutisch annehmbaren Salze von solchen Verbindungen, in denen $A = OR_6$ und darin $R_6$ Wasserstoff ist, mit Basen, wobei die mit «nieder» bezeichneten Reste höchstens 7 Kohlenstoffatome aufweisen.

Die Erfindung betrifft in erster Linie Verbindungen der allgemeinen Formel I, in den $R_1$ durch Niederalkyl, Niederalkoxy oder Halogen, insbesondere Fluor substituiertes oder vor allem unsubstituiertes Phenyl, $R_2$ primäres Niederalkyl, vor allem Methyl, $R_3$ und $R_5$ je Wasserstoff und $A = OR_6$ und darin $R_6$ Wasserstoff oder Niederalkyl bedeutet, und $R_4$ Methyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, sowie die pharmazeutisch annehmbaren Salze von solchen Verbindungen, in denen $A = OR_6$ und darin $R_6$ Wasserstoff ist, mit Basen, wie die den drei obengenannten Natriumsalzen zugrundeliegenden Carbonsäuren und ihre pharmazeutisch annehmbaren Salze mit Basen, wobei die mit «nieder» bezeichneten Reste höchstens 7 Kohlenstoffatome aufweisen.

Die neuen Verbindungen der allgemeinen Formel I

und Salze solcher Verbindungen werden in an sich bekannter Weise hergestellt, indem man

a) eine Verbindung der allgemeinen Formel II

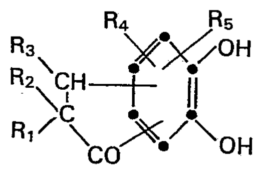

(II)

in welcher $R_1$, $R_2$, $R_3$, $R_4$ und $R_5$ die unter Formel I angegebene Bedeutung haben, oder ein Salz davon mit einer Verbindung der allgemeinen Formel III

Hal
>CH-CO-A (III)
Hal

in welcher Hal Halogen bedeutet und A die unter der Formel I angegebene Bedeutung hat, oder einem Salz einer solchen Verbindung, in der $A = OR_6$ und darin $R_6$ Wasserstoff bedeutet, umsetzt, oder

b) in einer Verbindung der allgemeinen Formel

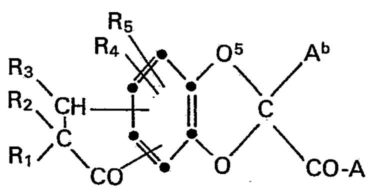

(IV)

in welcher $A^b$ Carboxy, Niederalkoxycarbonyl oder Acetyl bedeutet und $R_1$, $R_2$, $R_3$, $R_4$, $R_5$ und A die unter der Formel I angegebene Bedeutung haben, den Rest $A^b$ durch Wasserstoff ersetzt, oder

c) zur Herstellung einer Verbindung der allgemeinen Formel I, in welcher $A = OR_6$ und darin Wasserstoff bedeutet und $R_1$, $R_2$, $R_3$, $R_4$ und $R_5$ die unter der Formel I angegebene Bedeutung haben, oder eines Salzes dieser Verbindung in einer Verbindung der allgemeinen Formel V

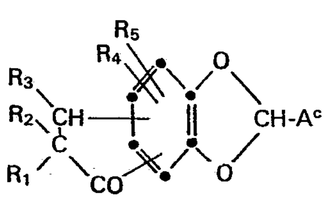

(V)

in welcher $A^c$ eine in die Carboxygruppe überführbare Gruppe bedeutet und $R_1$, $R_2$, $R_3$, $R_4$ und $R_5$ die unter der Formel I angegebene Bedeutung haben, die Gruppe $A^c$ in die Carboxygruppe in freier oder Salzform überführt, oder

d) zur Herstellung einer Verbindung der allgemeinen Formel I, in welcher A die unter der Formel I angegebene Bedeutung hat mit Ausnahme eines Restes $OR_6$, in welchem $R_6$ Wasserstoff bedeutet, und $R_1$, $R_2$, $R_3$, $R_4$ und $R_5$ die unter der Formel I angegebene Bedeutung haben, in einer Verbindung der allgemeinen Formel VI

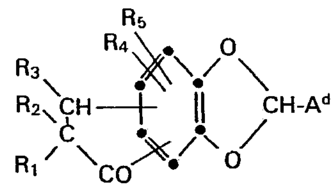

(VI)

in welcher $A^d$ einen in einen Rest $-CO-A_1$, in welchem $A_1$ die unter Formel I für A angegebene Bedeutung hat mit Ausnahme eines Restes $OR_6$, in welchem $R_6$ Wasserstoff bedeutet, überführbaren Rest bedeutet, und $R_1$, $R_2$, $R_3$, $R_4$ und $R_5$ die unter Formel I angegebene Bedeutung haben, den Rest A in den Rest $-CO-A_1$ überführt,
und gewünschtenfalls eine erhaltene Verbindung der allgemeinen Formel I in an sich bekannter Weise in eine andere Verbindung der allgemeinen Formel I überführt, und/oder eine als Racematgemisch erhaltene Verbindung der allgemeinen Formel I in die Racemate auftrennt und/oder eine als Racemat oder diastereomeres Antipodengemisch erhaltene Verbindung der allgemeinen Formel I in die optischen Antipoden zerlegt, und/oder eine erhaltene Verbindung der allgemeinen Formel I, in welcher $A = OR_6$ und darin $R_6$ Wasserstoff bedeutet, in ein Salz mit einer Base überführt oder eine solche Verbindungen aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

In den Ausgangsstoffen der allgemeinen Formel III ist Hal vorzugsweise Chlor oder Brom, kann aber auch Fluor oder Jod sein, wobei auch zwei unter sich verschiedene Halogenatome vorliegen können. Die Umsetzungen gemäss Verfahren a) werden vorzugsweise in unter den Reaktionsbedingungen inerten organischen Lösungsmitteln, beispielsweise in ätherartigen, wie z.B. Dibutyläther, 1,2-Dimethoxyäthan, Diäthylenglykoldimethyläther, Tetrahydrofuran oder Dioxan; alkoholartigen, wie z.B. Methanol, Äthanol, Isopropanol, Butanol, 2-Methoxyäthanol oder 2-Äthoxyäthanol; oder amidartigen, wie z.B. Dimethylformamid oder N,N,N',N',N'',N''-Hexamethylphosphorsäuretriamid; oder in Kohlenwasserstoffen, wie z.B. Petroläther, Cyclohexan, Benzol oder Toluol durchgeführt. Umsetzungen mit freien Verbindungen der allgemeinen Formel II wie auch mit freien Halogenessigsäuren der allgemeinen Formel III werden vorzugsweise in Gegenwart von basischen Stoffen durchgeführt. Als solche können beispielsweise organische oder anorganische Derivate von Alkali- oder Erdalkalimetallen, als organische Derivate z.B. Alkalimetall- oder Erdalkalimetallalkoxide, wie Natrium- oder Lithiummethoxid, -äthoxid, -n-butoxid oder -tert.-butoxid, bzw. Barium-methoxid, oder als anorganische Derivate z.B. entsprechende Hydroxide, wie Natrium-, Kalium- oder Calciumhydroxid, oder Carbonate, wie z.B. Natrium- oder Kaliumcarbonat, verwendet werden. Insbesondere Carbonate können in grösserem, z.B. bis fünffachem Überschuss eingesetzt werden. Bei Verwendung von Carbonaten können auch noch weitere organische Lösungsmittel, wie Niederalkanone, z.B. Aceton oder 2-Butanon, als genügend inert in Betracht kommen. Als Salze von Verbindungen der allgemeinen Formel II und von gegebenenfalls verwendeten, unter die allgemeine Formel III fallenden Dihalogenessigsäuren eignen sich beispielsweise entsprechende Alkalimetallsalze oder Erdalkalimetallsalze. Die Reaktionstemperaturen liegen beispielsweise zwischen Raumtemperatur und etwa 150°C und vorzugsweise zwischen etwa 70 und 120°C.

Die Ausgangsstoffe der allgemeinen Formel II sind ihrerseits neue Verbindungen, welche beispielsweise wie folgt herstellbar sind: Man kondensiert zunächst Veratrol, das entsprechend der Definition für $R_4$ und $R_5$ substituiert sein kann, mit einem durch Aryl oder Heteroaryl $R_1$ substituierten Acetylhalogenid nach Friedel-Crafts, z.B. mittels Aluminiumchlorid in 1,2-Dichloräthan bei Raumtemperatur, zum entsprechenden, gegebenenfalls gemäss der Bedeutung von $R_4$ und $R_5$ substituierten 1-(vic-Dimethoxyphenyl)-2-$R_1$-1-äthanon, von welcher Stoffgruppe einzelne Vertreter, wie das 1-(3,4-Dimethoxyphenyl)-2-phenyl-1-äthanon (3,4-Dimethoxy-desoxybenzoin) [vgl. S.F. Dyke et al., Tetrahedron 31, 1219-1222 (1975)] bekannt sind. In die 2-Stellung der erhaltenen Äthanone wird z.B. durch eine Modifikation der Mannichreaktion ein Niederalkylidenrest, z.B. zur Herstellung von Vorprodukten für Endstoffe mit Wasserstoff als Rest $R_3$ der Methylenrest, durch Umsetzung mit Bis-(dimethylamino)-methan in Gegenwart von Acetanhydrid, eingeführt. Insbesondere zur Herstellung von Homologen, d.h. Verbindungen mit einem anderen Niederalkylidenrest, z.B. dem Äthylidenrest, in gleicher Stellung kommen z.B. auch die Kondensation von gegebenenfalls definitionsgemäss substituierten Veratrol mit 2-$R_1$-2-niederalkenoylchlorid, z.B. dem bekannten 2-Phenylcrotonoylchlorid, sowie weitere an sich bekannte Verfahren zur Herstellung von Desoxybenzoinen in Frage. Die so erhaltenen, gegebenenfalls entsprechend $R_3$, $R_4$ und $R_5$ substituierten 1-(vic-Dimethoxyphenyl)-2--$R_1$-2-propen-1-one werden nun mittels einer starken Mineralsäure, z.B. Polyphosphorsäure, zu den entsprechenden 2,3-Dihydro-5,6-(oder 4,5)-dimethoxy-2-$R_1$-1H-inden-1-onen cyclisiert. In deren 2-Stellung wird gewünschtenfalls anschliessend in an sich bekannter Weise Niederalkyl $R_2$, z.B. durch Umsetzung mit einem Niederalkylhalogenid, wie Methyljodid, in einem zweiphasischen System aus einer konzentrierten wässrigen Lösung von Tetrabutylammoniumhydroxid oder -bromid und einem inerten organischen Lösungsmittel, z.B. Methylenchlorid, eingeführt. Schliesslich erhält man die Ausgangsstoffe der allgemeinen Formel II durch Spaltung der beiden Methoxygruppen in an sich bekannter Weise, z.B. durch Erhitzen mit 48%iger Bromwasserstoffsäure in wasserfreier Essigsäure oder durch Erhitzen mit Pyridinhydrochlorid. Einige Ausgangsstoffe der allgemeinen Formel III sind bekannt und weitere analog zu den bekannten Verbindungen herstellbar.

Zur Durchführung des Verfahrens b) erhitzt man beispielsweise einen Ausgangsstoff der allgemeinen Formel IV, in welchem $A^b$ Carboxy bedeutet und A wie auch $R_1$, $R_2$, $R_3$, $R_4$ und $R_5$ die unter Formel I angegebene Bedeutung haben, in An- oder Abwesenheit eines Katalysators, z.B. Kupferpulver, und/oder eines Lösungs- oder Verdünnungsmittels, wie z.B. o-Dichlorbenzol oder 1,2,3,4-Tetrahydronaphthalin, bis die mindestens annähernd äquimolare Menge Kohlendioxid freigesetzt ist. Ausgangsstoffe der allgemeinen Formel IV, in denen $A^b$ Carboxy und $A = OR_6$ und darin $R_6$ Wasserstoff bedeutet, werden beispielsweise durch Hydrolyse von entsprechenden Verbindungen, in denen $A = OR_6$ und darin $R_6$ Niederalkyl ist und als bzw. anstelle von $A^b$ Niederalkoxycarbonyl oder Cyano steht, in saurem oder alkalischem Medium, beispielsweise durch Erwärmen mit einer starken Mineralsäure in wässerigem oder wässerig-organischem, z.B. wässerig-niederalkanolischem Medium, bzw. mit der mindestens doppeltmolaren Menge eines Alkalimetallhydroxids, insbesondere Natrium- oder Kaliumhydroxids, z.B. in einem Niederalkanol, wie Methanol, Äthanol, Isopropanol oder n-Butanol, oder in einem Niederalkandiol oder Monoalkyläther desselben, z.B. Äthylen glykol, 2-Methoxyäthanol oder 2-Äthoxyäthanol hergestellt, wobei den genannten Lösungsmitteln gegebenenfalls Wasser im Volumenverhältnis von ca. 10 : 1 bis 1 : 2 zugefügt wird. Ferner kann als Reaktionsmedium auch Wasser oder z.B. ein Gemisch von Wasser mit wasserlöslichen, ätherartigen Lösungsmitteln wie Dioxan oder Tetrahydrofuran, verwendet werden.

Erfolgt die Hydrolyse in einer wasserhaltigen Mineralsäure, so kann die verfahrensgemässe Decarboxylierung daran anschliessend, d.h. im gleichen Medium und Arbeitsgang, durchgeführt werden.

Ausgangsstoffe der allgemeinen Formel IV, in denen $A^b$ Carboxy und A ein Rest entsprechend der Definition unter der Formel I ist mit Ausnahme eines Restes $OR_6$, in welchem $R_6$ Wasserstoff ist, kann man z.B. durch analoge Hydrolyse in alkalischem Medium von entsprechenden Verbindungen mit Niederalkoxycarbonyl als Rest $A^b$ unter Verwendung der etwa äquimolaren Menge eines Alkalimetallhydroxids anstelle der mindestens doppelmolaren Menge herstellen. Eine andere Möglichkeit zur Herstellung solcher Ausgangstoffe der allgemeinen Formel IV besteht in der Hydrogenolyse von entsprechenden Verbindungen, in denen sich anstelle von $A^b$ Benzyloxycarbonyl befindet.

Die Desalkoxycarbonylierung oder Deacetylierung von entsprechenden Ausgangsstoffen der allgemeinen Formel IV, d.h. solchen, in denen $A^b$ Niederalkoxycarbonyl oder Acetyl und A irgendeinen definitionsgemässen Rest bedeutet mit Ausnahme eines Restes $OR_6$, in welchem $R_6$ Wasserstoff bedeutet, erfolgt beispielsweise durch Umsetzung mit der etwa äquimolaren Menge eines Alkalimetallniederalkoxides in einem wasserfreien Niederalkanol, wobei, falls $A = OR_6$ und darin $R_6$ Niederalkyl ist, vorzugsweise dasselbe niedere Alkanol, z.B. Methanol, Äthanol, n-Butanol, als Komponente des Ausgangsesters und des Niederalkoxids wie auch als Reaktionsmedium gewählt wird. Man kann aber auch

durch Verwendung eines relativ höhersiedenden, mit dem als Esterkomponente vorliegenden Niederalkanol nicht identischen Alkanols als Reaktionsymedium und Abdestillieren eines Teiles davon gleichzeitig mit der definitionsgemässen Umsetzung eine Umesterung durchführen oder aber eine partielle Umesterung in Kauf nehmen, falls der als Reaktionsprodukt anfallende Ester der allgemeinen Formel I nicht direkt als Wirkstoff verwendet, sondern zur entsprechenden Säure hydrolysiert werden soll. Ferner kann als Reaktionsmedium anstelle eines niederen Alkanols z.B. auch ein inertes organisches Lösungsmittel, wie z.B. Benzol oder Toluol, verwendet werden. Die definitionsgemässe Umsetzung wird bei Raumtemperatur oder erhöhter Temperatur, z.B. bei Siedetemperatur des verwendeten Reaktionsmediums, durchgeführt. Gegebenenfalls kann der entstandene Ester der allgemeinen Formel I, wie bereits im Zusammenhang mit der Umesterung erwähnt, im gleichen Arbeitsgang zur entsprechenden Säure hydrolysiert werden, wenn man dem Reaktionsmedium Wasser zufügt.

Die Ausgangsstoffe der allgemeinen Formel IV, in denen $A^b$ Niederalkoxycarbonyl oder Acetyl ist, sowie die weiter oben genannten Vorprodukte zu Verbindungen der allgemeinen Formel IV mit Carboxy als Rest $A^b$, die als bzw. anstelle von $A^b$ Niederalkoxycarbonyl bzw. Cyano enthalten, können analog Verfahren a) durch Umsetzung von Verbindungen der allgemeinen Formel II mit geminalen Dihalogenverbindungen, die sich von solchen der allgemeinen Formel III durch das Vorliegen von Niederalkoxycarbonyl, Acetyl oder Cyano anstelle des neben beiden Halogenatomen befindlichen Wasserstoffatoms unterscheiden, in Gegenwart einer Base hergestellt werden.

Bei der Herstellung von Verbindungen der allgemeinen Formel I, in denen A den Rest $OR_6$ und darin $R_6$ Wasserstoff bedeutet, gemäss Verfahren c) kann die Umwandlung einer Gruppe $A^c$ in die Carboxygruppe in an sich bekannter Weise, insbesondere durch Hydrolyse in alkalischem oder saurem Medium erfolgen, wobei man im ersteren Falle auch direkt ein Salz erhalten kann. Ausgangsstoffe für die Hydrolyse sind zunächst solche Verbindungen der allgemeinen Formel I, in denen A kein Rest $OR_6$ ist, in welchem $R_6$ Wasserstoff bedeutet, besonders leicht hydrolysierbare unter ihnen, wie z.B. die Niederalkylester, im weiteren aber auch andere funktionelle Derivate der als Endstoffe gewünschten Carbonsäuren, wie z.B. Nitrile und Imidoester, insbesondere Imidoniederalkylester, von unter die allgemeine Formel I fallenden Carbonsäuren. Die Hydrolyse erfolgt beispielsweise in niederalkanolischen oder wässrig-niederalkanolischen Alkalihydroxidlösungen bei Raumtemperatur bis etwa 100°C bzw. Siedetemperatur des Reaktionsmediums. Niederalkylester, wie Methyl- oder Äthylester und andere leicht spaltbare Ester von unter die allgemeine Formel I fallenden Carbonsäuren können unter noch milderen Bedingungen, z.B. in Gegenwart von Kalium- oder Natriumcarbonat bei Raumtemperatur oder nötigenfalls schwach erhöhten Temperaturen von z.B. 40°C, in wässerig-organischem Medium, z.B. in dem beim Versetzen des bei der Umsetzung gemäss a) erhaltenen Reaktionsgemisches in einem mit Wasser mischbaren Lösungsmittel, wie z.B. 1,2-Dimethoxyäthan, mit Wasser hydrolysiert werden. Aus den dabei zunächst erhaltenen Alkalimetallsalzlösungen der unter die allgemeine Formel I fallenden Carbonsäuren kann man entweder durch Einengen bzw. Eindampfen und Umkristallisieren direkt das entsprechende reine Alkalisalz gewinnen oder zunächst die Carbonsäure freisetzen, anschliessend z.B. durch Umkristallisation reinigen und gewünschtenfalls wiederum in ein Salz mit einer geeigneten anorganischen oder organischen Base überführen. Funktionelle Derivate der unter die allgemeine Formel I fallenden Carbonsäure lassen sich ferner auch in saurem Medium, z.B. durch Erwärmen in einer mit Wasser verdünnen, z.B. 60-70%igen Schwefelsäure oder in niederalkanolisch-wässriger Salzsäure, in die freie Carbonsäure der allgemeinen Formel I überführen.

Die benötigten funktionellen Derivate von Carbonsäuren, d.h. Verbindungen der allgemeinen Formel V, welche unter die allgemeine Formel I fallen, werden z.B. gemäss Verfahren a) oder b), und andere funktionelle Derivate, wie z.B. Nitrile, analog zu diesen Verfahren hergestellt.

Ausgangsstoffe der allgemeinen Formel VI sind entsprechend dem in ihnen enthaltenen Rest $A^d$ beispielsweise Carbonsäuren, die auch unter die allgemeine Formel I fallen, weiter Carbonsäurehalogenide oder -anhydride, insbesondere gemischte Anhydride, aktivierte Ester, z.B. Cyanomethylester, sowie auch Niederalkylester, die, gegebenenfalls in Gegenwart von Kondensationsmitteln, mit Hydroxyverbindungen der allgemeinen Formel VII

$$R_6 - OH \qquad (VII)$$

oder Ammoniak bzw. Aminen der allgemeinen Formel VIII

$$HN{<}^{R_7}_{R_8} \qquad (VIII)$$

in welchen Formeln $R_6$ bzw. $R_7$ und $R_8$ die unter Formel I angegebene Bedeutung haben, umgesetzt werden können, oder Salze, insbesondere Alkalimetall- oder Erdalkalimetallsalze, der freien Carbonsäuren, die mit reaktionsfähigen Estern von Hydroxyverbindungen der allgemeinen Formel VII, wie Halogeniden oder organischen Sulfonsäureestern, z.B. Niederalkansulfonsäure- oder Arensulfonsäureestern, wie Methansulfonsäure- bzw. p-Toluolsulfonsäureestern, oder auch mit von Aminen der allgemeinen Formel VIII, deren Reste $R_7$ und $R_8$ von Wasserstoff verschieden sind, abgeleiteten Carbaminsäurehalogeniden, insbesondere -chloriden, umgesetzt werden können; weiter z.B. die zu Estern, insbesondere Niederalkylestern bzw. zu unsubstituierten Amiden hydrolysierbaren Imidoester, insbesondere Imidoniederalkylester, bzw. Nitrile. Freie Carbonsäuren können z.B. auch mit Diazoniederalkanen zu Niederalkylestern oder mit Isocyanaten, die von unter die allgemeine Formel VI fallenden primären Aminen abgeleitet sind, zu N-monosubstituierten Amiden umgesetzt werden.

Die Umsetzungen von freien Carbonsäuren mit Hydroxyverbindungen der allgemeinen Formel VII erfolgen vorteilhaft in Gegenwart eines sauren wasserabspaltenden Katalysators, wie einer Protonensäure, z.B. von Chlor- oder Bromwasserstoff-, Schwefel-, Phosphor- oder Borsäure, Benzolsulfon- oder Toluolsulfonsäure, oder einer Lewissäure, z.B. von Bortrifluorid-Ätherat, in einem Überschuss der eingesetzten Hydroxyverbindung und/oder in einem inerten Lösungsmittel, z.B. in einem Kohlenwasserstoff der Benzolreihe, wie Benzol oder Toluol, einem halogenierten Kohlenwasserstoff, wie Chloroform, Methylenchlorid oder Chlorbenzol, oder in einem ätherartigen Lösungsmittel, wie Tetrahydrofuran, erforderlichenfalls unter destillativer, z.B. azeotroper, Entfernung des bei der Reaktion freigesetzten Wassers. Weiter kann man die Umsetzungen auch in Gegenwart anderer wasserbindender Kondensationsmittel, z.B. von durch Kohlenwasserstoffreste substituierten Carbodiimiden, wie N,N'-Dicyclohexyl- oder N-Äthyl-N'-(3-dimethylaminopropyl)-carbodiimid, in inerten organischen Lösungsmitteln, z.B. den vorgenannten, durchführen. Halogenide und gemischte Anhydride werden beispielsweise in Gegenwart säurebindender Mittel, z.B. organischer, insbesondere tertiärer Stickstoffbasen, wie z.B. Triäthylamin, Äthyldiisopropylamin oder Pyridin, oder auch anorganischer Basen, z.B. von Alkalimetall- oder Erdalkalimetallhydroxiden oder -carbonaten, wie Natrium-, Kalium- oder Calciumhydroxid bzw. -carbonat, in inerten organischen Lösungsmitteln, z.B. den obengenannten, und nötigenfalls unter Erwärmen umgesetzt. Die Umsetzungen von reaktionsfähigen Estern von Carbonsäuren der allgemeinen Formel I, z.B. den Cyanomethylestern, mit Hydroxyverbindungen der allgemeinen Formel VII werden beispielsweise in einem gegenüber den Reaktionsteilnehmern inerten Lösungsmittel, z.B. in einem Kohlenwasserstoff wie Toluol oder Xylol, einem ätherartigen Lösungsmittel, wie Tetrahydrofuran oder Dioxan, oder bei mässigen Temperaturen auch einem Ester, wie Äthylacetat, im Temperaturbereich von etwa 0°C bis etwa 120°C, vorzugsweise bei Raumtemperatur bis etwa 60°C durchgeführt. Zu Umesterungen von Niederalkylestern von Carbonsäuren der allgemeinen Formel I verwendet man vorzugsweise Hydroxyverbindungen der allgemeinen Formel VII mit deutlich über demjenigen des veresterten Niederalkanols liegendem Siedepunkt und führt die Reaktion z.B. in einem Überschuss der Hydroxyverbindung und/oder einem inerten organischen, vorzugsweise ebenfalls deutlich über dem Niederalkanol siedenden Lösungsmittel, vorzugsweise in Gegenwart eines Katalysators, z.B. eines Alkalimetall-niederalkoxids, wie Natrium- oder Kalium-methoxid oder -äthoxid, in der Wärme und vorzugsweise unter Abdestillieren des freigesetzten Niederalkanols durch. Die Hydrolyse von Imidoestern, insbesondere Imidoniederalkylestern, von Carbonsäuren der allgemeinen Formel I erfolgt beispielsweise mittels wasserhaltiger Mineralsäure, wie Salzsäure oder Schwefelsäure, wobei man z.B. die bei der Addition von Chlorwasserstoff an Nitrile und Umsetzung mit wasserfreien Hydroxyverbindungen der allgemeinen Formel VII, insbesondere Niederalkanolen, erhaltenen Imidoester-hydrochloride nach Zusatz von Wasser direkt zu den entsprechenden Estern hydrolysieren kann, oder z.B. auch aus einem Gemisch von Nitril, Hydroxyverbindung und Schwefelsäure von geeignetem Wassergehalt ohne Isolierung des in situ entstandenen Imidoesters den entsprechenden Ester der allgemeinen Formel I erhalten kann.

Die Umsetzung von freien Carbonsäuren der allgemeinen Formel I mit Verbindungen der allgemeinen Formel VIII erfolgt beispielsweise in Gegenwart der obengenannten wasserbindenden Mittel und in den dort genannten inerten organischen Lösungsmitteln, man kann aber auch die aus den freien Carbonsäuren und den Verbindungen der allgemeinen Formel VIII zunächst gebildeten Ammoniumsalze durch Erhitzen, gegebenenfalls in einem geeigneten organischen Lösungsmittel von mittlerem oder höherem Siedepunkt, wie z.B. Xylol, Chlorbenzol oder 1,2,-3,4-Tetrahydronaphthalin, und destillative, gegebenenfalls azeotropische, Entfernung des bei der Reaktion freigesetzten Wassers, in Amide der allgemeinen Formel I überführen.

Als reaktionsfähige funktionelle Derivate von Carbonsäuren der allgemeinen Formel I zur Umsetzung mit Verbindungen der allgemeinen Formel VIII und als zugehörige Kondensationsmittel und Lösungsmittel kommen im wesentlichen dieselben in Betracht wie sie oben für Umsetzungen mit Hydroxyverbindungen der allgemeinen Formel VII angegeben wurden, mit dem Unterschied, dass man als säurebindende Mittel und gegebenenfalls einziges Reaktionsmedium anstelle anderer, d.h. tertiärer organischer Basen auch einen Überschuss an der umzusetzenden Verbindung der allgemeinen Formel VIII verwenden kann. Die als weitere Möglichkeit zur Bildung N-substituierter Amide erwähnte partielle Hydrolyse der entsprechenden Nitrile kann beispielsweise mittels wasserhaltiger Mineralsäuren, wie Salzsäure oder verdünnter Schwefelsäure bei Raumtemperatur oder mässig erhöhter Temperatur durchgeführt werden.

Die als Ausgangsstoffe für das Verfahren d) benötigten, von der allgemeinen Formel I umfassten freien Carbonsäuren, die zugleich der allgemeinen Formel I entsprechen, sind gemäss Verfahren a), b) und/oder c), und deren reaktionsfähige funktionelle Derivate z.B. aus den freien Carbonsäuren in an sich bekannter Weise herstellbar.

Erhaltene salzbildende Verbindungen der Formel I können in an sich bekannter Weise in Salze übergeführt werden, z.B. Verbindungen mit Hydroxy als Rest A mit entsprechenden Basen, wie z.B. Alkalimetallhydroxiden, in Salze mit Basen, oder solche von basischem Charakter in ihre Säureadditionssalze. Vorzugsweise werden pharmazeutisch annehmbare Salze hergestellt.

Erhaltene Salze können in an sich bekannter Weise in die freien Verbindungen umgewandelt werden, z.B. durch Behandeln mit einem sauren Reagens, wie einer Mineralsäure, bzw. mit einer Base, z.B. einer Alkalimetallhydroxidlösung, wie Natronlauge.

Die Verbindungen einschliesslich ihrer Salze können auch in Form ihrer Hydrate erhalten werden, oder ihre Kristalle können das zur Kristallisation verwendete Lösungsmittel einschliessen.

Infolge der engen Beziehung zwischen den neuen Verbindungen der allgemeinen Formel I, in welchem

A Hydroxy bedeutet, in freier Form und in Form ihrer Salze mit Basen, sowie von solchen Verbindungen, deren Rest $R_1$ basischen Charakter aufweist, in freier Form und in Form von Säureadditionssalzen, sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die neuen Verbindungen können, je nach der mindestens 2 betragenden Zahl der Asymmetriezentren, wie auch je nach der Wahl der Ausgangsstoffe, Verfahren und Arbeitsweisen, in Form von Gemischen von zwei oder mehreren Racematen, von reinen Racematen, von diastereomeren Antipodengemischen oder als reine optische Antipoden erhalten werden.

Erhaltene Racematgemische können aufgrund der physikalisch-chemischen Unterschiede der Bestandteile in bekannter Weise in die reinen Racemate bzw. Diastereomeren aufgetrennt werden, beispielsweise durch Chromatographie und/oder fraktionierte Kristallisation. Dank der guten Kristallisierbarkeit ist es meist leicht möglich, das eine von zwei Racematen als einheitliches Kristallisat zu gewinnen und gewünschtenfalls aus der Mutterlauge das andere Racemat zu isolieren. Erhaltene Racemate lassen sich nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes der allgemeinen Formel I mit einer mit der racemischen Säure Salze bildenden optisch aktiven Base, wie z.B. (+)-Dehydroabietylamin oder (+)- oder (—)-Ephedrin bzw. Umsetzung eines basischen Endstoffes der allgemeinen Formel I mit einer optisch aktiven Säure, wie z.B. (+)- oder (—)-Di-O,O'-(p-Toluoyl)-weinsäure und Trennung der auf diese Weise erhaltenen Salze, z.B. aufgrund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet. Beispielsweise kann man im Verfahren a) einen optischen Antipoden einer Verbindung der allgemeinen Formel II einsetzen und so als unmittelbares Reaktionsprodukt anstelle eines Gemisches von zwei diastereomeren Racematen ein Gemisch von zwei diastereomeren, sich nicht entsprechenden optischen Antipoden, d.h. ein diastereomeres Antipodengemisch erhalten, das sowohl mittels üblicher physikalischer Methoden als auch analog racemischen Antipodengemischen, d.h. unter Umwandlung in Salze, z.B. solche mit organischen Basen, wie der bereits genannten, oder in andere Derivate von optisch aktiven Verbindungen, z.B. Ester mit (+)- oder (—)-Menthol, zerlegt werden kann.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

Die Erfindung betrifft im weiteren pharmazeutische Präparate, die Verbindungen der allgemeinen Formel I als Wirkstoffe enthalten, sowie Verfahren zu ihrer Herstellung.

Bei den erfindungsgemässen pharmazeutischen Präparaten handelt es sich um solche zur enteralen, wie oralen oder rektalen, sowie zur parenteralen Verabreichung an Warmblüter. Die Dosierung des Wirkstoffs, der allein oder zusammen mit den üblichen Träger- und Hilfsmaterialien appliziert wird, hängt von den Warmblüterspezies, deren Alter und dem individuellen Zustand sowie der Applikationsweise ab. Die täglichen Dosen bewegen sich zwischen 0,3 und 30 mg/kg für Mammalien und liegen für solche von etwa 70 kg Gewicht je nach individuellem Zustand und Alter vorzugsweise zwischen 20 und 750 mg, insbesondere zwischen 50 und 500 mg. Entsprechende orale Doseneinheitsformen, z.B. Dragées oder Tabletten oder Kapseln, enthalten vorzugsweise 10 bis 250 mg, insbesondere 25 bis 150 mg eines erfindungsgemässen Wirkstoffes, d.h. einer Verbindung der allgemeinen Formel I oder eines pharmazeutisch annehmbaren Salzes einer zur Salzbildung befähigten Verbindung der allgemeinen Formel I zusammen mit pharmazeutischen Trägerstoffen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidin, und/oder, wenn erwünscht, Sprengmittel, wie die obgenannten Stärken, ferner Carboxymethylstärke, quervernetztes Polyvinylpyrrolidin, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Überzügen, Lösungen von

geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die Erfindung betrifft ebenfalls die Verwendung der neuen Verbindungen der Formel I und von pharmazeutisch annehmbaren Salzen von solchen als pharmakologisch aktive Verbindungen, insbesondere als Diuretika mit urikosurischer Zusatzwirkung, vorzugsweise in Form von pharmazeutischen Präparaten, in einem Verfahren zur prophylaktischen und/oder therapeutischen Behandlung des tierischen oder menschlichen Körpers, insbesondere zur Behandlung von Oedemen oder der Hypertension.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

*Beispiel 1*

25,43 g (0,10 Mol) 2,3-Dihydro-5,6-dihydroxy-2-methyl-2-phenyl-1H-inden-1-on und 15,7 g (12,3 ml, 0,10 Mol) Dichloressigsäure-äthylester werden mit 70 g (0,5 Mol) pulverisiertem wasserfreiem Kaliumcarbonat in 250 ml 1,2-Dimethoxyäthan bei kräftigem Rühren in Stickstoffatmosphäre 2 Stunden unter Rückfluss gekocht, wobei aus der anfänglichen Suspension eine dunkle Lösung entsteht, von der sich am Rande eine dunkle Schmiere abscheidet. Nach dem Erkalten gibt man 250 ml Wasser zu und rührt die dunkle Lösung eine Stunde bei 40°C. Hierauf wird das 1,2-Dimethoxyäthan im Vakuum abgedampft und die zurückbleibende wässrige Lösung durch allmähliche Zugabe von 6-n.Salzsäure kongosauer gestellt. Die ausgefallene Carbonsäure wird mit Äthylacetat extrahiert. Die Äthylacetatlösung extrahiert man ihrerseits mit 1-n.Natriumbicarbonatlösung, stellt letztere Lösung wiederum mit 6-n.Salzsäure kongosauer, nimmt die Carbonsäure wiederum in Äthylacetat auf und dampft die erhaltene Lösung ein. Die zurückbleibende Carbonsäure wird in 55 ml 2-n.Natronlauge gelöst, die Lösung mit Bleicherde versetzt und filtriert. Durch Abkühlen des Filtrates im Eisbad und Animpfen wird das Natriumsalz der 6,7-Dihydro-6-methyl-5-oxo-6-phenyl-5H-indeno[5,6-d]-1,3-dioxol-2-carbonsäure in feinen, schwer filtrierbaren Blättchen zur Kristallisation gebracht. Diese werden durch einen Zellstoffilter abfiltriert, mit wenig eiskaltem Wasser gewaschen und 20 Stunden bei Raumtemperatur unter 100 mbar getrocknet. Das so erhaltene Natriumsalz schmilzt oberhalb 50°C unter Zersetzung.

Formel der Säure:

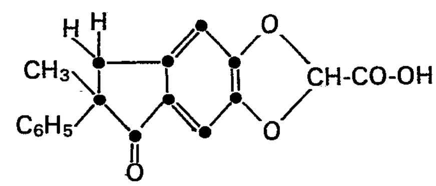

Das benötigte Indenonderivat wird die folgt hergestellt:

a) Zu einer Mischung von 25,6 g (0,1 Mol) 1-(3,4-Dimethoxyphenyl)-2-phenyl-1-äthanon [vgl. S.F. Dyke et al., Tetrahedron 31, 1219-1222 (1975)] und 50 ml (ca. 0,4 Mol) Bis-(dimethylamino)-methan werden unter Rühren bei Raumtemperatur innerhalb von 10 Minuten 50 ml (0,52 Mol) Acetanhydrid zugetropft, wobei man durch Kühlen mittels Eisbad dafür sorgt, dass die Temperatur des Reaktionsgemisches nicht weiter steigt, wenn sie 40°C erreicht hat. Hierauf wird die entstandene gelbliche Lösung noch eine Stunde gerührt, wobei man die Innentemperatur auf Raumtemperatur absinken lässt. Dann wird die Reaktionslösung unter kräftigem Rühren und Animpfen vorsichtig in ein Eis-Wasser-Gemisch gegossen, so dass eine gut filtrierbare Suspension entsteht. Das kristallisierte 1-(3,4-Dimethoxyphenyl)-2-phenyl-2-propen-1-on wird bei 10° abfiltriert und 18 Stunden unter 130 mbar bei 40° getrocknet, Smp. 74-75,5°. Die Substanz wird wegen geringer Beständigkeit möglichst rasch weiterverarbeitet.

b) Zu 135 ml (ca. 275 g) Polyphosphorsäure werden bei 95° unter Rühren innerhalb von 5 Minuten 13,4 g 1-(3,4-Dimethoxyphenyl)-2-phenyl-2-propen-1-on portionsweise zugegeben. Unter leichtem Selbsterwärmen auf etwa 100° entsteht eine hellbraune Lösung. Diese wird nach beendeter Zugabe noch eine halbe Stunde bei 95 bis 100° gerührt und dann auf Raumtemperatur abgekühlt. Unter Rühren und Eiskühlung werden 300 ml Wasser vorsichtig derart zugetropft, dass die Innentemperatur unterhalb 40° bleibt. Die entstandene grünliche Suspension wird genutscht, das Nutschgut mit Wasser gewaschen und hierauf eine Stunde bei 40° getrocknet. Das so erhaltene 2,3-Dihydro-5,6-dimethoxy-2--phenyl-1H-inden-1-on schmilzt bei 149-152° und nach Umkristallisation aus Aceton-Äthylacetat bei 154-155°.

c) Zu 330 ml einer 40%igen wässrigen Lösung von Tetrabutylammoniumhydroxid (ca. 0,5 Mol) lässt man unter Rühren bei Raumtemperatur innerhalb einer Stunde eine Lösung von 126,7 g (0,47 Mol) 2,3-Dihydro-5,6-dimethoxy-2-phenyl-1H-inden-1-on und 142 g (62,5 ml, 0,994 Mol) Methyljodid in 2000 ml Methylenchlorid zutropfen. Unter schwacher Erwärmung entsteht dabei eine gelbliche Suspension. Diese wird noch weitere 6 Stunden bei Raumtemperatur gerührt. Dann werden die Phasen getrennt, die organische Phase zweimal mit Wasser gewaschen, getrocknet, filtriert und eingedampft. Der Rückstand wird unter Erwärmen in 300 ml Äthanol gelöst. Hierbei und beim anschliessenden Kühlen kristallisiert das 2,3-Dihydro-5,6-dimethoxy-2-methyl-2-phenyl-1H-inden-1-on. Es wird abfiltriert, mit kaltem Gemisch von Äthanol und Petroläther (Siedebereich 40-65°) gewaschen und bei 40° unter ca. 130 mbar 18 Stunden getrocknet. Smp. 132-133°.

Aus der Mutterlauge kann durch Eindampfen und Zusatz von ca. 200 ml Äther das Tetrabutylammonium-jodid in Form von Kristallen gewonnen werden.

d) 5,6 g (0,020 Mol) 2,3-Dihydro-5,6-dimethyl--2-methyl-2-phenyl-1H-inden-1-on werden in einem Gemisch von 22,7 ml (0,2 Mol) 48%iger Bromwasserstoffsäure und 25 ml wasserfreier Essigsäure 5 Stunden auf 110° erhitzt, dann mit 2 ml einer 33%-igen Bromwasserstofflösung in Essigsäure versetzt und nochmals 8 Stunden auf 110° erhitzt. Nach dem Erkalten wird das Reaktionsgemisch auf Eiswasser gegossen und zweimal mnit Äther extrahiert. Die vereinigten Ätherlösungen werden zweimal mit Wasser, einmal mit wässriger 1-n.Natriumbicarbonatlösung und noch zweimal mit Wasser gewaschen, getrocknet, filtriert und eingedampft. Das zurückbleibende rohe 2,3-Dihydro-5,6-dihydroxy-2--methyl-2-phenyl-1H-inden-1-on kann direkt weiterverarbeitet werden.

*Beispiel 2*

26,83 g (0,10 Mol) 2,3-Dihydro-4,5-dihydroxy--2,7-dimethyl-2-phenyl-1H-inden-1-on und 15,7 g (12,3 ml, 0,10 Mol) Dichloressigsäure-äthylester werden mit 70 g (0,5 Mol) pulverisiertem wasserfreiem Kaliumcarbonat in 300 ml 1,2-Dimethoxyäthan unter kräftigem Rühren in Stickstoffatmosphäre 4 Stunden unter Rückfluss gekocht. Dann wird das Reaktionsgemisch auf 40° abgekühlt, mit 300 ml Wasser versetzt und eine Stunde bei 35-40° gerührt. Anschliessend wird das 1,2-Dimethoxyäthan im Rotationsverdampfer unter Vakuum abgedampft und die zurückbleibende Lösung in der Kälte durch vorsichtige Zugabe von 6-n.Salzsäure kongosauer gestellt. Die ausgefallene Säure wird mit Äthylacetat extrahiert, die Äthylacetatlösung zweimal mit Wasser gewaschen, getrocknet, filtriert und eingedampft. Eine Probe des als Schaum erstarrten Rückstandes wird aus Toluol umkristallisiert; die so erhaltene 7,8--Dihydro-5,7-dimethyl-6-oxo-7-phenyl-6H-indeno-[4,5-d]-1,3-dioxol-2-carbonsäure schmilzt bei 161-163° (nach Sintern ab 159°). Der übrige Rückstand wird in 40 ml 2-n.Natronlauge unter Erwärmen gelöst, die warme Lösung mit Bleicherde versetzt, in der Wärme filtriert und dann im Eisbad abgekühlt. Das als feine Nädelchen auskristallisierte Natriumsalz der 7,8-Dihydro-5,7-dimethyl-6-oxo-7-phenyl--6H-indeno[4,5-d]-1,3-dioxol-2-carbonsäure wird abfiltriert, mit eiskaltem Wasser gewaschen und unter 0,13 mbar 15 Stunden bei 30° getrocknet. Hierauf schmilzt es bei 187-190°. Formel:

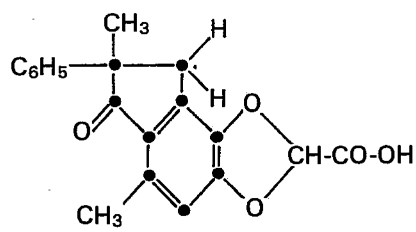

Durch Ansäuern der Mutterlauge, Extraktion mit Äther und Eindampfen im Vakuum erhält man die zur obigen Säure diastereomere Säure als amorphen Schaum.

Das benötigte Indenoderivat wird wie folgt hergestellt:

a) in eine Lösung von 48,1 g (0,5 Mol) 1,2-Dimethoxy-4-methylbenzol (Homobrenzcatechindimethyläther bzw. 4-Methylveratrol) in 750 ml 1,2-Dichloräthan werden unter Rühren 80,0 g (0,6 Mol) Aluminiumchlorid portionsweise eingetragen, wobei man durch Kühlen mit einem Eisbad die Temperatur bei höchstens 20° hält. Dann werden bei 15-20° innerhalb einer Stunde 85,0 g (ca. 73 ml, 0,55 Mol) Phenylacetylchlorid zugetropft, wobei mässige Chlorwasserstoffentwicklung eintritt und das Aluminiumchlorid aufgelöst wird.

Hierauf wird das Reaktionsgemisch noch 6 Stunden bei Raumtemperatur gerührt und anschliessend auf 3000 ml Eis-Wasser-Gemisch gegossen. Die entstandenen Schichten werden getrennt und die organische Phase nacheinander mit 2-n.Salzsäure, zweimal mit Wasser, dann mit 1-n.Natriumbicarbonatlösung und noch zweimal mit Wasser gewaschen, getrocknet, filtriert und eingedampft. Der zum Teil kristallisierte Rückstand wird aus Äther-Petroläther (Siedebereich 40-65°) vollständig zur Kristallisation gebracht, wobei das 1-(4,5-Dimethoxy-2-methylphenyl)-2-phenyl-1-äthanon vom Smp. 45-48° erhalten wird.

b) Zu einer Mischung von 135,0 g (0,50 Mol) 1--(4,5-Dimethoxy-2-phenyl-1-äthanon und 250 ml Bis-(dimethylamino)-methan werden unter Rühren bei Raumtemperatur innerhalb von 20 Minuten 240 ml Acetanhydrid zugetropft, wobei man durch Kühlen mittels Eisbad den Temperaturanstieg bei 40° hält. Man rührt das Reaktionsgemisch noch eine Stunde bei 40° und giesst es dann auf 2 kg Eis-

Wasser-Gemisch, wobei das Rohprodukt bereits kristallisiert. Nach 15 Minuten Rühren bei 15° wird das Rohprodukt abfiltriert, das Filtergut mit kaltem Wasser gewaschen und hierauf bei etwa 100 mbar und 30° 18 Stunden getrocknet, wobei man das 1-(4,5--Dimethoxy-2-methylphenyl)-2-phenyl-2-propen-1--on vom Smp. 57-59° erhält.

c) In 700 ml auf 90° erwärmte Polyphosphorsäure streut man ohne Wärmezufuhr unter Rühren innerhalb 2 bis 3 Minuten 70,0 g (0,25 Mol des kristallisierten Produktes von b) ein. Dabei steigt die Temperatur auf etwa 95° und es entsteht ein violettes Gemisch. Dieses erhitzt man sofort auf 105° und rührt es noch 15 Minuten bei 105-110°. Dann kühlt man es auf 40° ab und fügt unter Rühren 1000 ml Toluol und 300 ml Methylenchlorid und hierauf 3 kg Eis zu und rührt bei 10-20° weiter, bis eine homogene Emulsion entstanden ist. Dann werden die Schichten getrennt und die organische Phase einmal mit gesättigter Natriumbicarbonatlösung und zweimal mit Wasser gewaschen. Die als organische Phase erhaltene Lösung des 2,3-Dihydro-4,5-dimethoxy-7-methyl-2-phenyl-1H-inden-1-on kann direkt für die nächste Stufe verwendet werden.

d) Zu einer Mischung von 96,5 g Tetrabutylammoniumbromid und 60 ml konz. (etwa 10-n.)Natronlauge lässt man bei Raumtemperatur unter Rühren und Kühlen mittels Wasserbad innerhalb von 25 Minuten die gemäss c) erhaltene Lösung von etwa 0,25 Mol des gemäss c) entstandenen Indenonderivats, der zuvor 42,6 g (18,7 ml, 0,3 Mol) Methyljodid zugefügt wurden, zulaufen und rührt das Gemisch, dessen Temperatur anfänglich leicht steigt, noch weitere 4 Stunden ohne Wärmezufuhr. Dann trennt man die organische Phase ab, wäscht sie dreimal mit Wasser, trocknet sie über Natriumsulfat, filtriert sie und dampft sie auf etwa 300 ml ein. Bei 20° wird die ausgefallene Mischung von Tetrabutylammoniumbromid und -jodid abfiltriert und mit Toluol gewaschen. Das Filtrat dampft man zur Trockne ein und versetzt den Rückstand mit etwa der zehnfachen Menge Äther. Nach einer Stunde Stehenlassen im Eisbad werden die gelblichen Kristalle abfiltriert. Das so erhaltene 2,3-Dihydro-4,5-dimethoxy-2,7-dimethyl-2-phenyl-1H-inden-1-on schmilzt bei 94-95°.

e) Ein Gemisch von 29,6 g (0,10 Mol) des nach d) erhaltenen Indenonderivates, 115 ml 48%iger Bromwasserstoffsäure, 100 ml wasserfreier Essigsäure und 25 ml einer 33%igen Lösung von Bromwasserstoff in Essigsäure wird 8 Stunden in Stickstoffatmosphäre unter Rückfluss gekocht und nach dem Erkalten auf 1500 g Eis-Wasser-Gemisch gegossen und zweimal mit Äther extrahiert. Die vereinigten Ätherphasen werden mit Wasser, dann mit 1-n.Natriumbicarbonatlösung und noch zweimal mit Wasser gewaschen, mit Natriumsulfat getrocknet, filtriert und eingedampft. Der bräunliche Rückstand kristallisiert bei längerem Stehen.

Er wird unter Erwärmen in Äther gelöst, mit Bleicherde versetzt und filtriert. Das Filtrat wird mit Cyclohexan versetzt, der Äther im Rotationsverdampfer teilweise abgedampft und die Lösung mit der gewünschten Substanz angeimpft, wobei Kristallisation eintritt. Nach Zugabe von wenig Petroläther dampft man den Äther weiter ab und filtriert dann das auskristallisierte, 2,3-Dihydro-4,5-dihydroxy-2,7-dimethyl-2-phenyl-1H-inden-1-on ab. Es schmilzt bei 160-163°.

*Beispiel 3*

Analog Beispiel 2 erhält man ausgehend von 27,21 g (0,10 Mol) 2,3-Dihydro-5,6-dihydroxy-2-(4-fluorphenyl)-1-methyl-1H-inden-1-on und 15,7 g (0,10 Mol) Dichloressigsäure-äthylester das nach längerem Stehenlassen seiner wässrigen Lösung in der Kälte als feine Blättchen kristallisierende Natriumsalz der 6,7-Dihydro-6-(4-fluorphenyl)-6-methyl-5-oxo--5H-indeno[5,6-d]-1,3-dioxol-2-carbonsäure. Dieses Salz wird nach Umkristallisation aus Wasser-Aceton unter Abdestillieren des Acetons als Trihydrat erhalten, das oberhalb 50° schmilzt.

Das als Ausgangsstoff verwendete Indenonderivat wird wie folgt hergestellt:

a) 160 g (1,2 Mol) pulverisiertes Aluminiumchlorid werden in 1500 ml 1,2-Dichloräthan suspendiert und die Suspension auf 10° abgekühlt. Dann gibt man unter Rühren 139,0 g (1,0 Mol) Veratrol in einem Mal zu, wobei die Temperatur auf 28° ansteigt und ein grosser Teil des Aluminiumchlorids in Lösung geht. Hierauf tropft man bei 20° unter Rühren innerhalb einer Stunde 190,9 g (1,1 Mol) (4-Fluorphenyl)--acetylchlorid zu. Unter mässiger Chlorwasserstoffentwicklung und schwach exothermer Reaktion färbt sich die Reaktionslösung dunkel. Sie wird anschliessend noch $1^1/_2$ Stunden bei Raumtemperatur gerührt und dann auf 2 kg Eis-Wasser-Gemisch gegossen und mit 50 ml konz. Salzsäure versetzt. Die organische Phase wird abgetrennt und einmal mit 2-n.Salzsäure, zweimal mit Wasser, einmal mit 1-n.Natriumbicarbonatlösung und zweimal mit Wasser gewaschen. Die wässrige Phase wird einmal mit Chloroform extrahiert und der nach Abdampfen des Chloroforms erhaltene Eindampfrückstand zur organischen Phase gegeben. Letztere wird nach Filtration im Vakuum eingedampft und der noch warme, ölige Rückstand mit etwa 300 ml Cyclohexan versetzt und kräftig geschüttelt, damit eine gut filtrierbare Kristallsuspension entsteht. Nach Kühlen wird diese filtriert und das Filtergut mit Cyclohexan gewaschen. Das so erhaltene 1-(3,4-Dimethoxyphenyl)-2-(4--fluorphenyl)-1-äthanon schmilzt bei 102-104° nach Sintern ab 100°.

b) Analog Beispiel 2 b) erhält man unter Verwendung von 109,7 g (0,40 Mol) 1-(3,4-Dimethoxyphenyl)-2-(4-fluorphenyl)-1-äthanon das 1-(3,4-Dimethoxyphenyl)-2-(4-fluorphenyl)-2-propen-1-on, das beim Giessen des Reaktionsgemisches auf Eis-Wasser-Gemisch als farblose Kristalle anfällt, welche nach 16 Stunden Trocknen bei 40° unter ca. 130 mbar bei 77-78° schmelzen.

c) Analog Beispiel 2 c) erhält man unter Verwendung von 85,9 g (0,30 Mol) 1-(3,4-Dimethoxyphenyl)-2-(4-fluorphenyl)-2-propen-1-on das 2,3-Dihydro-5,6-dimethoxy-2-(4-fluorphenyl)-1H-inden-1--on, welches beim Versetzen des Reaktionsgemisches mit Wasser kristallisiert. Die Kristalle werden mit Wasser gewaschen und getrocknet und dann in 2500 ml Methylenchlorid gelöst. Die Lösung wird von abgeschiedenem schwarzgrünem Harz dekantiert mit Bleicherde versetzt filtriert, auf etwa 500 ml

eingeengt und die entstandene Suspension mit etwa 500 ml Äthylacetat versetzt. Nach Abdestillieren des restlichen Methylenchlorids kühlt und filtriert man die Kristalle des obigen Reaktionsproduktes ab und wäscht sie mit Äthylacetat; Smp. 189 - 191°.

d) Analog Beispiel 2 d) erhält man unter Verwendung von 47,7 g (0,167 Mol) 2,3-Dihydro-5,6-dimethoxy-2-(4-fluorphenyl)-1H-inden-1-on das rohe 2,3-Dihydro-5,6-dimethoxy-2-(4-fluorphenyl)-2-methyl-1H-inden-1-on. Der Eindampfrückstand der organischen Phase wird, statt mit Äther, mit 120 ml Äthanol versetzt, wobei nach Kratzen das Reaktionsprodukt als feine Nädelchen auskristallisiert. Nach Kühlen wird es abfiltriert mit Äthanol-Äther gewaschen und 16 Stunden bei 40° unter 130 mbar getrocknet, worauf es bei 100 - 101° schmilzt.

e) Analog Beispiel 2 e) erhält man unter Verwendung von 30,0 g (0,10 Mol) des Produktes von d) das rohe 2,3-Dihydro-5,6-dihydroxy-2-(4-fluorphenyl)-2-methyl-1H-inden-1-on als erstarrten Schaum. Das Produkt kann direkt weiterverarbeitet werden.

*Beispiel 4*

Analog Beispiel 2 erhält man unter Verwendung von 28,64 g (0,10 Mol) 2,3-Dihydro-4,5-dihydroxy-2,7-dimethyl-2-(4-fluorphenyl)-1H-inden-1-on das Natriumsalz der 7,8-Dihydro-5,7-dimethyl-7-(4-fluorphenyl)-6-oxo-6H-indeno[4,5-d]-1,3-dioxol-2-carbonsäure als Monohydrat vom Smp. 188 - 190°.

Das benötigte Indenonderivat wird wie folgt hergestellt.

a) Eine Lösung von 1682 g (1,0 Mol) 1,2-Dimethoxy-4-methylbenzol in 1500 ml 1,2-Dichloräthan wird in einem Eisbad vorgekühlt und unter Rühren portionenweise mit 160 g (1,2 Mol) pulverisiertem Aluminiumchlorid versetzt. Dann werden unter Rühren bei 15 - 20° innerhalb 30 Minuten 190 g (1,1 Mol) (4-Fluorphenyl)-acetylchlorid zugetropft, wobei mässige Chlorwasserstoffentwicklung eintritt und das Aluminiumchlorid aufgelöst wird. Nach weiteren 5 Stunden Rühren bei Raumtemperatur wird das Reaktionsgemisch auf etwa 5 kg Eis-Wasser-Gemisch gegossen. Die Phasen werden im Scheidetrichter getrennt und die Wasserphase zweimal mit Methylenchlorid extrahiert und der Eindampfrückstand der Methylenchloridlösung wird zur organischen Phase gegeben. Letztere wird nacheinander mit 2-n.Salzsäure, zweimal mit Wasser, zweimal mit gesättigter Natriumbicarbonatlösung mit anschliessender Filtration über Bleicherde, und wieder zweimal mit Wasser gewaschen, über Natriumsulfat getrocknet, filtriert und eingedampft. Der noch warme Rückstand wird in Äther gelöst und die Ätherlösung durch Eindampfen auf ca. 700 ml eingeengt. Beim Abkühlen kristallisiert das 2-(4-Fluorphenyl)-1-(4,5-dimethoxy-2-methyl-phenyl)-1-äthanon. Es wird abgenutscht und mit kaltem Äther nachgewaschen; Smp. 96 - 97°.

b) Analog Beispiel 1 b) erhält man unter Verwendung von 115,3 g (0,40 Mol) 2-(4-Fluorphenyl)-1-(4,5-Dimethoxy-2-methylphenyl)-1-äthanon das rohe 2-(4-Fluorphenyl)-1-(4,5-dimethoxy-2-methylphenyl)-2-propen-1-on, das direkt weiterverarbeitet wird.

c) Analog Beispiel 2 c) erhält man unter Verwendung des Rohproduktes von b) (115 g, annähernd 0,4 Mol) das rohe 2,3-Dihydro-4,5-dimethoxy-2-(4-fluorphenyl)-7-methyl-1H-inden-1-on.

d) Analog Beispiel 2 d) erhält man unter Verwendung von 50,1 g (0,167 Mol) des nach c) erhaltenen Indenonderivates das 2,3-Dihydro-4,5-dimethoxy-2,7-dimethyl-2-(4-fluorphenyl)-1H-inden-1-on als hell-sandfarbenes Pulver vom Smp. 102 - 104°.

e) Analog Beispiel 2 e) erhält man unter Verwendung von 31,44 g (0,10 Mol) des Reaktionsproduktes von d) das 2,3-Dihydro-4,5-dihydroxy-2,7-dimethyl-2-(4-fluorphenyl)-1H-inden-1-on als hellbraunes Pulver vom Smp. 189 - 190°.

*Beispiel 5*

Durch Umsetzung von 43,0 g (0,15 Mol) 2,3-Dihydro-4,5-dihydroxy-2,7-dimethyl-2-(2-fluorphenyl)-1H-inden-1-on [analog Beispielen 4a), 1b), 2c), 2d) und 2e) hergestellt] mit 23,8 g (0,15 Mol) Dichloressigsäureäthylester in Gegenwart von 105 g (0,75 Mol) pulverisiertem wasserfreiem Kaliumcarbonat in 430 ml 1,2-Dimethoxyäthan erhält man analog Beispiel 2 rohe 7,8-Dihydro-5,7-dimethyl-7-(2-fluorphenyl)-6-oxo-6H-indeno[4,5-d]-1,3-dioxol-2-carbonsäure als Eindampfrückstand der Äthylacetatlösung. Die rohe Säure kann z.B. wie folgt gereinigt weden: Man löst den Eindampfrückstand in 500 ml 1-n.Natronlauge, gibt wässrige Calciumchloridlösung zu, bis die Fällung nur noch schwach zunimmt, und filtriert den Niederschlag über Bleicherde. Das Filtrat wird mit 2-n.Salzsäure angesäuert und mit Äthylacetat extrahiert. Die Äthylacetatlösung wird wiederum eingedampft, der Rückstand in 2-n. Natronlauge gelöst, die Lösung mit 2-n.Salzsäure angesäuert und 2 Stunden kräftig gerührt, der Niederschlag abfiltriert, mit Wasser gewaschen und 40 Stunden bei 40° unter 130 mbar getrocknet. Die so erhaltene, spröde, amorphe Substanz schmilzt oberhalb 90° und ergibt für die gewünschte Carbonsäure zutreffende Analysenresultate.

*Beispiel 6*

6,50 g (0,062 Mol) 7,8-Dihydro-5,7-dimethyl-6-oxo-7-phenyl-6H-indeno[4,5-d]-1,3-dioxol-2-carbonsäure (in üblicher Weise freigesetzt aus dem in Beispiel 2 beschriebenen Natriumsalz) werden in 130 ml wasserfreiem Äthanol gelöst, die Lösung mit 0,1 ml einer 5-n.Chlorwasserstofflösung in Äther versetzt und eine Stunde unter Rückfluss gekocht. Dann wird das Reaktionsgemisch eingedampft, der Rückstand in Äther aufgenommen und die ätherische Lösung einmal mit 1-n.Natriumbicarbonatlösung und einmal mit Wasser gewaschen, über Natriumsulfat getrocknet, filtriert und eingedampft. Der bereits kristallisierte Rückstand wird aus Äthanol umkristallisiert, wobei man den 7,8-Dihydro-5,7-dimethyl-6-oxo-7-phenyl-6H-indeno[4,5-d]-1,3-dioxol-2-carbonsäureäthylester als farblose Nädelchen vom Smp. 96 - 97° erhält. Dieser diastereomere Ester ergibt im NMR-Spektrum folgende Signale:

1,34 ppm (3H t), 1,63 ppm (3H s), 2,59 ppm (3H s), 3,14 ppm (1H d), 3,52 ppm (1H d), 4,34 ppm (2H q), 6,38 ppm (1H s), 6,76 ppm (1H s), ca. 7,27 ppm (5H m).

Die aus der Mutterlauge des Natriumsalzes obiger Säure des Beispiels 2 freigesetzte und dort als Schaum isolierte diastereomere Säure kann ganz analog zu obigem Verfahren in den diastereomeren Äthylester übergeführt werden, der im Gegensatz zur freien Säure aus Äthanol kristallisiert und umkristallisiert werden kann und dann bei 93 bis 94° schmilzt. Im NMR-Spektrum ergibt dieser diastereomere Ester folgende Signale:

1,37 ppm (3H t), 1,65 ppm (2H s), 2,61 ppm (3H s), 3,20 ppm (1H d), 3,48 ppm (1H d), 4,34 (2H q), 6,39 ppm (1H s), 6,75 ppm (1H s), ca. 7,27 ppm (5H m).

*Beispiel 7*

a) Eine Lösung von 19,9 g (61,36 mMol) der nach Beispiel 2 erhaltenen racemischen 7,8-Dihydro-5,7-dimethyl-6-oxo-7-phenyl-6H-inden[4,5-d]-1,3-dioxol-2-carbonsäure vom Smp. 161 - 163°, 8,76 g (30,7 mMol) (+)-Dehydroabietylamin und 3,1 g (30,6 mMol) Triäthylamin in einer Mischung von 85 ml Methanol und 8,5 ml Wasser wird mehrere Stunden bei Raumtemperatur und anschliessend unter Kühlen mit Eis stehengelassen und hierauf das entstandene Dehydroabietylamin-Salz abfiltriert, welches nach zwei Umkristallisationen aus der 20fachen Menge einer Mischung aus 9 Teilen Methanol und einem Teil Wasser folgendes Schmelzverhalten zeigt:

A) Bei langsamem Aufheizen ≪ 5°/min resultiert ein Smp. von 138 - 139°

B) Beim Einsetzen in den auf 120° vorgeheizten Schmelzpunktbestimmungsapparat und raschem Weiterheizen mit ca. 5°/min resultiert ein Smp. von 178 - 180°, vorher tritt eine nematische Phase auf.

Setzt man von obigem Dehydroabietylamin-Salz auf bekannte Art die rechtsdrehende Säure frei, so erhält man diese als amorphen Schaum, welcher folgende Drehungswerte zeigt:

$[\alpha]_D^{20}$ + 47° (c = 1%, Aceton)

$[\alpha]_{436}^{20}$ + 193°

b) Aus den in der Mutterlauge obiger Trennung verbleibenen Salzen (vorwiegend Triäthylamin-Salz) wird in üblicher Weise wieder die Säure freigesetzt und isoliert und hierauf in Acetonitril als Lösungsmittel mit einem Äquivalent (—)-Ephedrin versetzt. Das nach mehrstündigem Stehenlassen und anschliessendem Eiskühlen abfiltrierte (—)-Ephedrin-Salz zeigt nach dreimaligem Umkristallisieren aus Acetonitril einen Smp. von 166 - 167°. Setzt man aus diesem Salz die Säure frei, erhält man einen amorphen Schaum mit folgenden Drehungswerten:

$[\alpha]_D^{20}$ + 45° (c = 1%, Aceton)

$[\alpha]_{436}^{20}$ + 190°

c) Die Trennung kann auch durchgeführt werden, indem man die racemische Säure sinngemäss zuerst mit (—)-Ephedrin und Triäthylamin in Acetonitril behandelt, das (—)-Ephedrin-Salz der links drehenden Säure abtrennt und aus den verbleibenden Mutterlaugen die angereicherte rechtsdrehende Säure mit (+)-Dehydroabietylamin in das entsprechende Salz überführt und letzteres durch Umkristallisation reinigt.

*Beispiel 8*

a) Zu einer bei 80° unter Stickstoffatmosphäre gerührten Mischung von 17,3 g Dichloressigsäure in 72 ml wässriger 5-n.Kalilauge und 0,3 g Tri-($C_8$-$C_{10}$-alkyl)-methylammoniumchlorid wird innerhalb von einer Stunde die Lösung von (+)-2,3-Dihydro-4,5-dihydroxy-2,7-dimethyl-2-phenyl-1H-inden-1-on in 35,5 ml 2-n.Kalilauge zugetropft. Man rührt das Reaktionsgemisch anschliessend 8 Stunden bei 80°, dann kühlt man es ab, säuert es mit 6-n.Salzsäure an und extrahiert die Säuren mit Äther. Der Ätherextrakt wird eingedampft und der Rückstand wird in 150 ml abs. Äthanol gelöst. Man setzt 0,5 ml ätherische 5-n. Chlorwasserstofflösung zu und kocht die Lösung eine Stunde unter Rückfluss, dann dampft man sie im Vakuum zur Trockne ein und nimmt den Rückstand wieder in Äther auf. Die Ätherlösung wird einmal mit verdünnter wässriger Natriumbicarbonatlösung und einmal mit Wasser gewaschen, anschliessend über Natriumsulfat getrocknet, filtriert und eingedampft. Der rohe Eindampfrückstand wird mit Hilfe eines Lösungsmittelgemisches von Chloroform (10 Teile), Hexan (10 Teile) und Äthylacetat (1 Teil) über ein Bett von 140 g Kieselgel filtriert und das so gereinigte Filtrat wieder zur Trockne eingedampft. Der Eindampfrückstand von 13,0 g wird mit 20 ml wässriger 2-n.Natronlauge versetzt und auf dem Wasserbad erwärmt, bis Lösung eintritt. Nach einer Stunde Stehenlassen bei Raumtemperatur wird die Lösung mit 2-n.Salzsäure sauer gestellt und die ausfallenden Säuren werden mit Äther extrahiert. Beim Eindampfen des Ätherextraktes im Vakuum erhält man die rohen Säuren als fast farblosen amorphen, spröden Schaum, bestehend aus den beiden diastereomeren Antipoden der 7,8-Dihydro-5,7-dimethyl-6-oxo-7-phenyl-6H-indeno[4,5-d]-1,3-dioxol-2-carbonsäure.

b) 11,1 g (34,2 mMol) des gemäss a) erhaltenen Säuregemisches werden mit einer Lösung von 4,88 g (17,1 mMol) (+)-Dehydroabietylamin und 1,72 g (17,1 mMol) Triäthylamin in 50 ml Methanol versetzt. Nach einigen Stunden Stehenlassen im Eisbad kristallisiert das Dehydroabietylaminsalz des einen Diastereomeren in feinen Nädelchen aus und kann durch Filtration entfernt werden. (Das Salz ist identisch in allen Eigenschaften mit dem gemäss Beispiel 7a) erhaltenen (+)-Dehydroabietylaminsalz). Das Filtrat wird zur Trockene eingedampft und aus dem Rückstand die Säure freigesetzt. Man erhält 6,9 g amorphen Schaum, zu etwa 90% bestehend aus dem stark rechtsdrehenden Diastereomeren der 7,8-Dihydro-5,7-dimethyl-6-oxo-7-phenyl-6H-indeno-[4,5-d]-1,3-dioxol-2-carbonsäure mit den folgenden Drehungswerten:

$[\alpha]_D^{20}$ + 166° (c = 1%, Aceton)

$[\alpha]_{436}^{20}$ + 478°

c) Zur weiteren Abtrennung von diastereomeren Verunreinigungen wird die gemäss b) erhaltene Säure nach einer bekannten Veresterungsmethode, z.B.: Chem. Ber. 95, Seite 1284 ff (1962), H.A. Staab und A. Mannschreck, mit (—)-Menthol in den Mentholester übergeführt und dieser durch Mitteldruckflüssigchromatographie gereinigt. Der Ester der stark rechtsdrehenden Form mit (—)-Menthol zeigt einen Smp. von 108 - 109° (Umkrist. aus Hexan).

d) 0,5 g des nach c) gewonnenen reinen Menthylesters werden in einer Mischung von 2 ml 1-n.Natronlauge, 5 ml Wasser und 20 ml Methanol zwei Stunden bei 60°C gerührt. Zum Aufarbeiten wird das Methanol im Vakuum abgedampft und der Rückstand mit Wasser verdünnt. Die Wasserphase wird zweimal mit Äther ausgezogen. Anschliessend stellt man die Wasserphase mit verdünnter Salzsäure sauer und extrahiert die ausgefällte Säure mit Äther. Man trocknet die Ätherlösung über Natriumsulfat, filtriert sie und dampft im Rotationsverdampfer unter Vakuum zur Trockene ein. Die stark rechtsdrehende Form der 7,8-Dihydro-5,7-dimethyl-6-oxo-7-phenyl-6H-indeno[4,5-d]-1,3-dioxol-2-carbonsäure bleibt als farbloser amorpher Schaum zurück und zeigt in diesem reinen Zustand folgende Drehungswerte:

$[\alpha]_D^{20}$ + 213° (c = 1%, Aceton)

$[\alpha]_{436}^{20}$ + 604°

e) Durch sinngemäss analoges Vorgehen kann man aus der gemäss i) gewonnenen linksdrehenden Phenoxyessigsäure analog zu den Schritten k) und a) bis d) die stark linksdrehende Form der 7,8-Dihydro-5,7-dimethyl-6-oxo-7-phenyl-6H-indeno[4,5-d]-1,3-dioxol-2-carbonsäure gewinnen.

Der optisch aktive Ausgangsstoff zu Stufe a) kann wie folgt erhalten werden:

f) 141,7 g (0,478 Mol) des nach Beispiel 2d) erhaltenen 2,3-Dihydro-4,5-dimethoxy-2,7-dimethyl-2-phenyl-1H-inden-1-on werden mit einer Mischung von 275 ml wässeriger 48%iger Bromwasserstoffsäure, 475 ml Eisessig und 72 ml HBr 33%-igem Bromwasserstoff in Eisessig 45 Minuten unter leichtem Rückfluss gekocht und dann auf 3000 ml einer Eis-Wasser-Mischung gegossen. Man extrahiert das ausgefallene Produkt zweimal mit Äther und wäscht einmal mit Wasser, einmal mit verdünnter Natriumbicarbonatlösung und wieder zweimal mit Wasser. Man trocknet die Ätherphasen über Natriumsulfat, filtriert sie und dampft sie im Rotationsverdampfer unter Vakuum zur Trockene ein. Der rohe Rückstand von 135 g braunen Kristallen wird in 250 ml Äthylacetat warm gelöst und filtriert. Dann setzt man 500 ml Petroläther vom Siedebereich 40-60° zu. Beim Stehenlassen kristallisiert das Produkt aus und kann filtriert und getrocknet werden. Man erhält auf diese Art das 2,3-Dihydro-2,7-dimethyl-4-hydroxy-5-methoxy-2-phenyl-1H-inden-1-on vom Smp. 136-139°.

g) 42,3 g (0,15 Mol) der nach e) erhaltenen 4-Hydroxyverbindung werden mit 28 g wasserfreiem, pulverisiertem Kaliumcarbonat und 32,3 g (0,19 Mol) Bromessigsäureäthylester zusammen mit 400 ml Dimethylformamid 1/2 Stunde bei 60° gerührt. Man versetzt anschliessend die entstandene Suspension mit 400 ml Wasser und 80 ml konz. wässeriger Natronlauge und erhitzt die entstandene Lösung eine Stunde auf 90°. Die jetzt blassgelbe Lösung wird mit 400 g Eis versetzt und mit konz. Salzsäure sauergestellt und das ausgefällte Produkt mit Äther extrahiert. Der Ätherextrakt wird über Natriumsulfat getrocknet, filtriert und eingeengt, wobei das Reaktionsprodukt kristallin auszufallen beginnt. Zur Vervollständigung der Kristallisation kühlt man im Eisbad und filtriert dann die Kristalle ab, wobei man die 2-(2,3-Dihydro-2,7-dimethyl-5-methoxy-2-phenyl-1H-inden-4-yloxy)-essigsäure vom Smp. 151-152° erhält.

h) Man löst 92,6 g (0,272 Mol) der nach g) erhaltenen racemischen Säure zusammen mit 13,8 g (0,136 Mol) Triäthylamin und 16,5 g (0,136 Mol) S-(—)-1-Phenyl-äthylamin in 300 ml Acetonitril. Das bei Raumtemperatur kristallisierende Salz mit S-(—)-1-Phenyläthylamin wird abfiltriert und mehrmals aus Acetonitril umkristallisiert, bis sein Schmelzpunkt von anfänglich 142-144° auf 160-161° angestiegen ist. Setzt man aus diesem Salz nun auf bekannte Art die rechtsdrehende 2-(2,3-Dihydro-2,7-dimethyl-5-methoxy-2-phenyl-1H-inden-4-yloxy)-essigsäure frei, so erhält man einen amorphen Schaum mit folgenden Drehungswerten:

$[\alpha]_D^{20}$ + 103° (c = 1%, Aceton)

$[\alpha]_{436}^{20}$ +316°

i) Durch analoges Vorgehen erhält man beim Einsatz von R(+)-1-Phenyläthylamin die linksdrehende 2-(2,3-Dihydro-2,7-dimethyl-5-methoxy-2-phenyl-1H-inden-4-yloxy)-essigsäure mit den folgenden Drehungswerten:

$[\alpha]_D^{20}$ —106° (c = 1%, Aceton)

$[\alpha]_{436}^{20}$ —326°

k) 15,2 g (44,7 mMol) der nach h) erhaltenen rechtsdrehenden Phenoxyessigsäure werden mit 80 g Pyridin-Hydrochlorid vermischt und in ein auf 200° vorgeheiztes Bad eingetaucht. Die so entstehende Schmelze wird für 4 1/2 Stunden bei gleicher Temperatur gerührt, dann giesst man die noch heisse Schmelze auf 500 g Eis, welchem man vorher 50 ml 6-n.Salzsäure zugesetzt hatte. Das ausfallende Produkt wird mit Äther extrahiert. Die Ätherextrakte wäscht man zweimal mit Wasser, trocknet über Natriumsulfat, filtriert und dampft zur Trockne ein. Man erhält ein in Form von sandfarbenem Schaum, rechtsdrehendes 2,3-Dihydro-4,5-dihydroxy-2,7-dimethyl-2-phenyl-1H-inden-1-on mit den Drehungswerten:

$[\alpha]_D^{20}$ +98° (c = 1%, Aceton)

$[\alpha]_{436}^{20}$ +304°

*Beispiel 9*

6,48 g (0,020 Mol) 7,8-Dihydro-5,7-dimethyl-6-oxo-7-phenyl-6H-indeno[4,5-d]-1,3-dioxol-2-carbonsäure werden in 10,0 ml reiner wässeriger 2-n.Kalilauge gelöst, die Lösung sofort filtriert und ca. 14 Stunden stehengelassen. Der entstandene Kristallkuchen wird aufgebrochen und 3 Stunden im Eisbad gekühlt. Die Kristalle werden abfiltriert, mit kaltem Wasser gewaschen und unter ca. 130 mbar bei 40° unter Luftzufuhr getrocknet. Das erhaltene Kaliumsalz der obigen Säure schmilzt bei 158-160° und enthält gemäss Analyse eine äquimolare Menge Kristallwasser.

*Beispiel 10*

4,86 g (0,015 Mol) 7,8-Dihydro-5,7-dimethyl-6-oxo-7-phenyl-6H-indeno[4,5-d]-1,3-dioxol-2-carbonsäure werden in 15,0 ml einer 1-n.Lösung von 2,2',2''-Nitrilotris-äthanol (Triäthanolamin) in Acetonitril unter Erwärmen gelöst, die Lösung sofort filtriert und ca. 14 Stunden stehengelassen. Der harte Kristallkuchen wird aufgebrochen und 3 Stunden im Eisbad gekühlt. Die hygroskopischen Kristalle werden abfiltriert, mit wenig kalten Acetonitril gewaschen und hierauf in wenig, d.h. ca. 12 ml warmem Wasser gelöst, die Lösung bei Raumtemperatur angeimpft und 2 Stunden im Eisbad gekühlt. Die so entstandenen groben Kristalle werden abfiltriert, mit kaltem Wasser gewaschen und unter 130 mbar bei 40° unter Luftzufuhr getrocknet. Das erhaltene N,N,N-Tris-(2-hydroxyäthyl-ammoniumsalz der obengenannten Säure schmilzt bei 140-142°.

*Beispiel 11*

6,48 g (0,020 Mol) 7,8-Dihydro-5,7-dimethyl-6--oxo-7-phenyl-6H-indeno[4,5-d]-1,3-dioxol-2-carbonsäure und 2,42 g (0,020 Mol) 2-Amino-2-(hydroxymethyl)-1,3-propandiol werden in 16 ml Wasser unter Erwärmen gelöst, die Lösung filtriert und ca. 14 Stunden stehengelassen. Der entstandene Kristallkuchen wird aufgebrochen und 3 Stunden im Eisbad gekühlt. Die Kristalle werden abfiltriert, mit kaltem Wasserr gewaschen und unter 130 mbar bei 40° unter Luftzufuhr getrocknet. Das erhaltene N-[2-Hydroxy-1,1-bis-(hydroxymethyl)-äthyl]-ammoniumsalz der obengenannten Säure schmilzt bei 155 bis 157°.

*Beispiel 12*

Tabletten enthaltend 100 mg Wirkstoff können beispielsweise in folgender Zusammensetzung hergestellt werden:

| Zusammensetzung | pro Tablette |
|---|---|
| Natriumsalz der 7,8-Dihydro-5,7-dimethyl-6-oxo-7-phenyl-6H-indeno[4,5-d]--1,3-dioxol-2-carbonsäure | 100 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 250 mg |

*Herstellung*

Der Wirkstoff wird mit Milchzucker, einem Teil der Weizenstärke und mit kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist. Die Masse wird durch ein Sieb von ca. 3 mm Maschenweite getrocknet und das trockene Granulat nochmals durch ein Sieb getrieben. Darauf werden die restliche Weizenstärke, Talk und Magnesiumstearat zugemischt. Die erhaltene Mischung wird zu Tabletten von je 250 mg mit Bruchkerbe(n) verpresst.

*Beispiel 13*

Um 1000 Kapseln mit je 100 mg Wirkstoffgehalt herzustellen, mischt man 100 g 7,8-Dihydro-5,7-dimethyl-6-oxo-7-phenyl-6H-indeno[4,5-d]-1,3-dioxol-2-carbonsäure mit 173,0 g Lactose, befeuchtet die Mischung gleichmässig mit einer wässrigen Lösung von 2,0 g Gelatine und granuliert sie durch ein geeignetes Sieb (z.B. Sieb III nach Ph. Helv. V.). Das Granulat mischt man mit 10,0 g getrockneter Maisstärke und 15,0 g Talk und füllt es gleichmässig in 1000 Hartgelatinekapseln der Grösse 1.

Anstelle des vorstehend verwendeten Wirkstoffes kann man in den Beispielen 12 und 13 auch eine Verbindung der allgemeinen Formel I oder ein Salz einer zur Salzbildung befähigten Verbindung der allgemeinen Formel I, z.B. einen der in den Beispielen 1 und 3 bis 11 beschriebenen Stoffe, verwenden.

**Patentansprüche für die Vertragsstaaten:**
BE, CH, DE, FR, GB, IT, LI, LU, NL, SE

1. Dioxaheterocyclische Verbindungen der allgemeinen Formel I

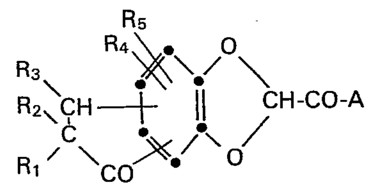

(I)

in denen $R_1$ durch Niederalkyl, Niederalkoxy oder Halogen substituiertes oder unsubstituiertes Phenyl oder Pyridyl, $R_2$ primäres Niederalkyl, $R_3$ und $R_4$ unabhängig voneinander Wasserstoff oder Niederalkyl, $R_5$ Wasserstoff bedeutet und A den Rest $-O-R_6$, worin $R_6$ für Wasserstoff oder einen unsubstituierten oder durch Niederalkoxy oder Halogen substituierten Alkylrest mit höchstens 12 C-Atomen, 2- oder 3-Niederalkenyl, 2-Niederalkinyl, Phenylniederalkyl oder Cinnamyl, in welchen der Phenylrest in gleicher Weise wie unter $R_1$ substituiert sein kann, steht, oder den Rest $-N\begin{matrix}R_7\\R_8\end{matrix}$, worin $R_7$ und $R_8$ unabhängig voneinander für Wasserstoff oder Niederalkyl, oder unter sich verbunden und zusammen mit dem anliegenden Stickstoffatom für gegebenenfalls niederalkylsubstituiertes Tetra-bis Hexamethylenimino oder 4-Morpholinyl stehen, bedeutet, und der angegebene zweiwertige Rest entweder in 4,5- oder in 5,6-Stellung und entsprechend $R_4$ und $R_5$ in den Stellungen 6 und 7 bzw. 4 und 7 des Benzodioxol-Molekülteils stehen, als Racematgemische, Racemate, diastereomere Antipodengemische oder optische Antipoden, sowie Salze von Verbindungen der allgemeinen Formel I, in denen $A = OR_6$ und darin $R_6$ Wasserstoff bedeutet, mit Basen, wobei die mit «nieder» bezeichneten Reste höchstens 7 Kohlenstoffatome aufweisen.

2. Verbindungen der im Anspruch 1 angegebenen allgemeinen Formel I, in denen $R_1$ durch Niederalkyl, Niederalkoxy oder Halogen substituiertes oder unsubstituiertes Phenyl oder Pyridyl, $R_2$ primäres Niederalkyl, $R_3$ Wasserstoff oder Niederalkyl, $R_5$ Wasserstoff und $A = OR_6$ und darin $R_6$ Wasserstoff oder Niederalkyl bedeutet, und $R_4$ Niederalkyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist, mit Basen, wobei die mit «nieder» bezeichneten Reste höchstens 7 Kohlenstoffatome aufweisen.

3. Verbindungen der im Anspruch 1 angegebenen allgemeinen Formel I, in denen $R_1$ durch Niederalkyl, Niederalkoxy oder Halogen substituiertes oder unsubstituiertes Phenyl, $R_2$ primäres Niederalkyl, $R_3$ und $R_5$ je Wasserstoff und $A = OR_6$ und darin $R_6$ Wasserstoff oder Niederalkyl bedeutet, und $R_4$ Niederalkyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist, mit Basen, wobei die mit «nieder» bezeichneten Reste höchstens 7 Kohlenstoffatome aufweisen.

4. Verbindungen der im Anspruch 1 angegebenen allgemeinen Formel I, in denen $R_1$ durch Niederalkyl, Niederalkoxy oder Halogen substituiertes oder unsubstituiertes Phenyl, $R_2$ primäres Niederalkyl, $R_3$ und $R_5$ je Wasserstoff und $A = OR_6$ und darin $R_6$ Wasserstoff oder Niederalkyl bedeutet, und $R_4$ Methyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist, mit Basen, wobei die mit «nieder» bezeichneten Reste höchstens 7 Kohlenstoffatome aufweisen.

5. Verbindungen der im Anspruch 1 angegebenen allgemeinen Formel I, in denen $R_1$ durch Niederalkyl, Niederalkoxy oder Fluor substituiertes oder unsubstituiertes Phenyl oder Pyridyl, $R_2$ Methyl, $R_3$ und $R_5$ je Wasserstoff und $A = OR_6$ und darin $R_6$ Wasserstoff oder Niederalkyl bedeutet, und $R_4$ Methyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist mit Basen, wobei die mit «nieder» bezeichneten Reste höchstens 7 Kohlenstoffatome aufweisen.

6. Die 7,8-Dihydro-5,7-dimethyl-6-oxo-7-phenyl-6H-indeno[4,5-d]-1,3-dioxol-2-carbonsäure als Racematgemisch, Racemat, diastereomeres Antipodengemisch oder optische Antipoden, und ihre Salze mit Basen.

7. Das Racemat der 7,8-Dihydro-5,7-dimethyl-6-oxo-7-phenyl-6H-indeno[4,5-d]-1,3-dioxol-2-carbonsäure vom Smp. 161-163°C und ihre Salze mit Basen.

8. Die 7,8-Dihydro-5,7-dimethyl-7-(2-fluorphenyl)-6-oxo-6H-indeno[4,5-d]-1,3-dioxo-2-carbonsäure und ihre Salze mit Basen.

9. Die 6,7-Dihydro-6-methyl-5-oxo-6-phenyl-5H-indeno[4,5-d]-1,4-dioxol-2-carbonsäure und ihre Salze mit Basen.

10. Die 6,7-Dihydro-6-(4-fluorphenyl)-6-methyl-5-oxo-5H-indeno[4,5-d]-1,3-dioxol-2-carbonsäure und ihre Salze mit Basen.

11. Die 7,8-Dihydro-5,7-dimethyl-7-(4-fluorphenyl)-6-oxo-6H-indeno[4,5-d]-1,3-dioxol-2-carbonsäure, deren Natriumsalz-Monohydrat bei 188 bis 190°C schmilzt, und ihre Salze mit Basen.

12. Das Racemat des 7,8-Dihydro-5,7-dimethyl-6-oxo-7-phenyl-6H-indeno[4,5-d]-1,3-dioxo-2-carbonsäure-äthylester vom Smp. 96-97°C.

13. Das Racemat des 7,8-Dihydro-5,7-dimethyl-6-oxo-7-phenyl-6H-indeno[4,5-d]-1,3-dioxol-2-carbonsäure-äthylester vom Smp. 93-94°C.

14. Pharmazeutische Präparate, enthaltend eine Verbindung gemäss einem der Ansprüche 1-13 oder ein pharmazeutisch annehmbares Salz einer zur Salzbildung befähigten Verbindung gemäss einem der Ansprüche 1 bis 11.

15. Verbindungen gemäss einem der Ansprüche 1-13 oder pharmazeutisch annehmbare Salze von zur Salzbildung befähigten Verbindungen gemäss einem der Ansprüche 1-11 zur Anwendung in einem Verfahren zur prophylaktischen und/oder therapeutischen Behandlung des tierischen oder menschlichen Körpers.

16. Verbindungen gemäss einem der Ansprüche 1-13 und pharmazeutisch annehmbare Salze von zur Salzbildung befähigten Verbindungen gemäss einem der Ansprüche 1-11 zur Verwendung als Diuretika mit urikosurischer Zusatzwirkung.

17. Verfahren zur Herstellung von Arzneimitteln, dadurch gekennzeichnet, dass man Verbindungen gemäss einem der Ansprüche 1 bis 13 oder pharmazeutisch annehmbare Salze von zur Salzbildung befähigten Verbindungen gemäss einem der Ansprüche 1-11 mit üblichen Zusätzen mischt.

18. Verfahren zur Herstellung dioxaheterocyclischer Verbindungen der im Anspruch 1 angegebenen allgemeinen Formel I, in welcher $R_1$, $R_2$, $R_3$, $R_4$ und A die dort definierte Bedeutung haben, und von Salzen derselben, dadurch gekennzeichnet, dass man

a) eine Verbindung der allgemeinen Formel II

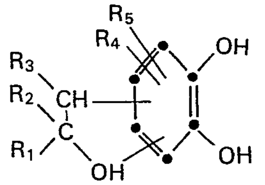

(II)

in welcher $R_1$, $R_2$, $R_3$, $R_4$ und $R_5$ die im Anspruch 1 angegebene Bedeutung haben, oder ein Salz davon mit einer Verbindung der allgemeinen Formel III

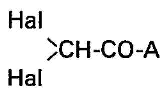

(III)

in welcher Hal Halogen bedeutet und A die im Anspruch 1 angegebene Bedeutung hat, oder einem Salz einer solchen Verbindung, in der $A = OR_6$ und darin $R_6$ Wasserstoff bedeutet, umsetzt, oder

b) in einer Verbindung der allgemeinen Formel

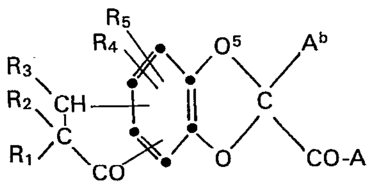

(IV)

in welcher $A^b$ Carboxy, Niederalkoxycarbonyl oder Acetyl bedeutet und $R_1$, $R_2$, $R_3$, $R_4$, $R_5$ und A die im Anspruch 1 angegebene Bedeutung haben, den Rest $A^b$ durch Wasserstoff ersetzt, oder

c) zur Herstellung einer Verbindung der allgemeinen Formel I, in welcher $A = OR_6$ und darin $R_6$ Wasserstoff bedeutet und $R_1$, $R_2$, $R_3$, $R_4$ und $R_5$ die im Anspruch 1 angegebene Bedeutung haben, oder eines Salzes dieser Verbindung in einer Verbindung der allgemeinen Formel V

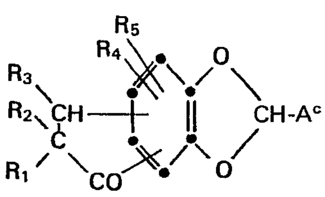

(V)

in welcher $A^c$ eine in die Carboxygruppe überführbare Gruppe bedeutet und $R_1$, $R_2$, $R_3$, $R_4$ und $R_5$ die im Anspruch 1 angegebene Bedeutung haben, die Gruppe $A^c$ in die Carboxygruppe in freier oder Salzform überführt, oder

d) zur Herstellung einer Verbindung der allgemeinen Formel I, in welcher A die im Anspruch 1 angegebene Bedeutung hat mit Ausnahme eines Restes $OR_6$, in welchem $R_6$ Wasserstoff bedeutet, und $R_1$, $R_2$, $R_3$, $R_4$ und $R_5$ die im Anspruch 1 angegebene Bedeutung haben, in einer Verbindung der allgemeinen Formel VI

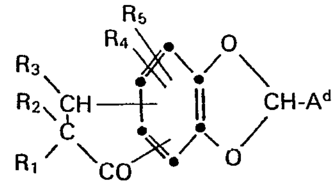

(VI)

in welcher $A^d$ einen in einen Rest $-CO-A_1$, in welchem $A_1$ die im Anspruch 1 für A angegebene Bedeutung hat mit Ausnahme eines Restes $OR_6$, in welchem $R_6$ Wasserstoff bedeutet, überführbaren Rest bedeutet, und $R_1$, $R_2$, $R_3$, $R_4$ und $R_5$ die im Anspruch 1 angegebene Bedeutung haben, den Rest $A^d$ in den Rest $-CO-A_1$ überführt,
und gewünschtenfalls eine erhaltene Verbindung der allgemeinen Formel I in an sich bekannter Weise in eine andere Verbindung der allgemeinen Formel I überführt, und/oder eine als Racematgemisch erhaltene Verbindung der allgemeinen Formel I in die Racemate auftrennt und/oder eine als Racemat oder diastereomeres Antipodengemisch erhaltene Verbindung der allgemeinen Formel I in die optischen Antipoden zerlegt, und/oder eine erhaltene Verbindung der allgemeinen Formel I, in welcher $A = OR_6$ und darin $R_6$ Wasserstoff bedeutet, in ein Salz mit einer Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

**Patentansprüche für den Vertragsstaat:** AT

1. Verfahren zur Herstellung dioxaheterocyclischer Verbindungen der allgemeinen Formel I

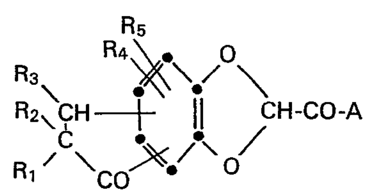

(I)

in denen $R_1$ durch Niederalkyl, Niederalkoxy oder Halogen substituiertes oder unsubstituiertes Phenyl oder Pyridyl, $R_2$ primäres Niederalkyl, $R_3$ und $R_4$ unabhängig voneinander Wasserstoff oder Niederalkyl, $R_5$ Wasserstoff bedeutet und A den Rest $-O-R_6$, worin $R_6$ für Wasserstoff oder einen unsubstituierten oder durch Niederalkoxy oder Halogen substituierten Alkylrest mit höchstens 12 C-Atomen, 2- oder 3-Niederalkenyl, 2-Niederalkinyl, Phenylniederalkyl oder Cinnamyl, in welchen der Phenylrest in gleicher Weise wie unter $R_1$ substituiert sein kann, steht, oder den Rest $-N<^{R_7}_{R_8}$ , unabhängig voneinander für

Wasserstoff oder Niederalkyl, oder unter sich verbunden und zusammen mit dem anliegenden Stickstoffatom für gegebenenfalls niederalkylsubstituiertes Tetra-bis Hexamethylenimino oder 4-Morpholinyl stehen, bedeutet, und der angegebene zweiwertige Rest entweder in 4,5- oder in 5,6-Stellung und entsprechend $R_4$ und $R_5$ in den Stellungen 6 und 7 bzw. 4 und 7 des Benzodioxol-Molekülteils stehen, und von Salzen derselben, wobei die mit «nieder» bezeichneten Reste höchstens 7 Kohlenstoffatome aufweisen, dadurch gekennzeichnet, dass man

a) eine Verbindung der allgemeinen Formel II

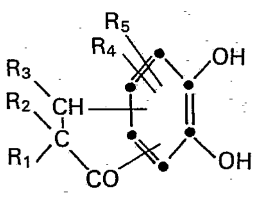

(II)

in welcher $R_1$, $R_2$, $R_3$, $R_4$ und $R_5$ die unter Formel I angegebene Bedeutung haben, oder ein Salz davon mit einer Verbindung der allgemeinen Formel III

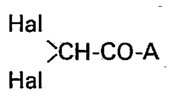

(III)

in welcher Hal Halogen bedeutet und A die unter Formel I angegebene Bedeutung hat, oder einem Salz einer solchen Verbindung, in der $A = OR_6$ und darin $R_6$ Wasserstoff bedeutet, umsetzt, oder

b) in einer Verbindung der allgemeinen Formel

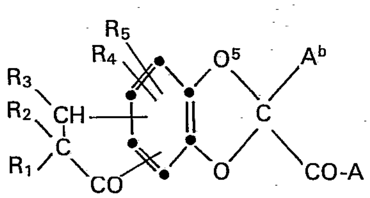

(IV)

in welcher $A^b$ Carboxy, Niederalkoxycarbonyl oder Acetyl bedeutet und $R_1$, $R_2$, $R_3$, $R_4$, $R_5$ und A die unter Formel I angegebene Bedeutung haben, den Rest $A^b$ durch Wasserstoff ersetzt, oder

c) zur Herstellung einer Verbindung der allgemeinen Formel I, in welcher $A = OR_6$ und darin $R_6$ Wasserstoff bedeutet und $R_1$, $R_2$, $R_3$, $R_4$ und $R_5$ die unter Formel I angegebene Bedeutung haben, oder eines Salzes dieser Verbindung in einer Verbindung der allgemeinen Formel V

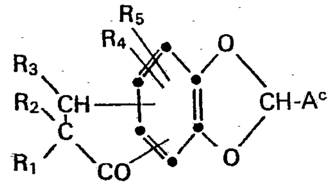

(V)

in welcher $A^c$ eine in die Carboxygruppe überführbare Gruppe bedeutet und $R_1$, $R_2$, $R_3$, $R_4$ und $R_5$ die unter Formel I angegebene Bedeutung haben, die Gruppe $A^c$ in die Carboxygruppe in freier oder Salzform überführt, oder

d) zur Herstellung einer Verbindung der allgemeinen Formel I, in welcher A die unter Formel I angegebene Bedeutung hat mit Ausnahme eines Restes $OR_6$, in welchem $R_6$ Wasserstoff bedeutet, und $R_1$, $R_2$, $R_3$, $R_4$ und $R_5$ die unter Formel I angegebene Bedeutung haben, in einer Verbindung der allgemeinen Formel VI

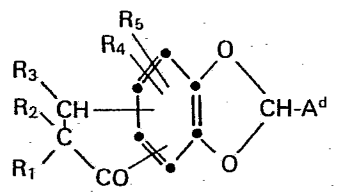

(VI)

in welcher $A^d$ einen in einen Rest $-CO-A_1$, in welchem $A_1$ die unter Formel I für A angegebene Bedeutung hat mit Ausnahme eines Restes $OR_6$, in welchem $R_6$ Wasserstoff bedeutet, überführbaren Rest bedeutet, und $R_1$, $R_2$, $R_3$, $R_4$ und $R_5$ die im unter Formel I angegebene Bedeutung haben, den Rest $A^d$ in den Rest $-CO-A_1$ überführt, und gewünschtenfalls eine erhaltene Verbindung der allgemeinen Formel I in an sich bekannter Weise in eine andere Verbindung der allgemeinen Formel I überführt, und/oder eine als Racematgemisch erhaltene Verbindung der allgemeinen Formel I in die Racemate auftrennt und/oder eine als Racemat oder diastereomeres Antipodengemisch erhaltene Verbindung der allgemeinen Formel I in die optischen Antipoden zerlegt, und/oder eine erhaltene Verbindung der allgemeinen Formel I, in welcher $A = OR_6$ und darin $R_6$ Wasserstoff bedeutet, in ein Salz mit einer Base überführt oder eine solche Verbindung aus einem erhaltenen Salz freisetzt.

2. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Halogen substituiertes oder unsubstituiertes Phenyl oder Pyridyl, $R_2$ primäres Niederalkyl, $R_3$ Wasserstoff oder Niederalkyl, $R_5$ Wasserstoff und $A = OR_6$ und darin $R_6$ Wasserstoff oder Niederalkyl bedeutet, und $R_4$ Niederalkyl in 5-Stellung des Tricyclus gebunden bedeutet und zugleich der angegebene zweiwertige Rest

in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist, mit Basen herstellt, wobei die mit «nieder» bezeichneten Reste höchstens 7 Kohlenstoffatome aufweisen.

3. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Halogen substituiertes oder unsubstituiertes Phenyl, $R_2$ primäres Niederalkyl, $R_3$ und $R_5$ je Wasserstoff und $A = OR_6$ und darin $R_6$ Wasserstoff oder Niederalkyl bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist, mit Basen herstellt, wobei die mit «nieder» bezeichneten Reste höchstens 7 Kohlenstoffatome aufweisen.

4. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Halogen substituiertes oder unsubstituiertes Phenyl, $R_2$ primäres Niederalkyl, $R_3$ und $R_5$ je Wasserstoff und $A = OR_6$ und darin $R_6$ Wasserstoff oder Niederalkyl bedeutet, und $R_4$ Methyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist, mit Basen herstellt, wobei die mit «nieder» bezeichneten Reste höchstens 7 Kohlenstoffatome aufweisen.

5. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Fluor substituiertes oder unsubstituiertes Phenyl, $R_2$ Methyl, $R_3$ und $R_5$ je Wasserstoff und $A = OR_6$ und darin $R_6$ Wasserstoff oder Niederalkyl bedeutet, und $R_4$ Methyl in 5-Stellung des Tricyclus gebunden bedeutet und zugleich der angegebene zweiwertige Rest in 4,5-Stellung des Benzodioxol-Molekülteils steht, oder $R_4$ Wasserstoff bedeutet und zugleich der angegebene zweiwertige Rest in 5,6-Stellung des Benzodioxol-Molekülteils steht, als Racematgemische, Racemate, diastereomere Antipodengemische oder optische Antipoden, sowie die pharmazeutisch annehmbaren Salze von solchen Verbindungen, in denen $A = OR_6$ und darin $R_6$ Wasserstoff ist, mit Basen herstellt, wobei die mit «nieder» bezeichneten Reste höchstens 7 Kohlenstoffatome aufweisen.

6. Verfahren nach Anspruch 1, dadurch gekennzeichnet, dass man die 7,8-Dihydro-5,7-dimethyl-6-oxo-7-phenyl-6H-indeno[4,5-d]-1,3-dioxol-2-carbonsäure als Racematgemisch, Racemat, diastereomeres Antipodengemisch oder optische Antipoden, und ihre Salze mit Basen herstellt.

7. Verfahren nach Anspruch 1, dadurch gekennzeichnet, dass man die 7,8-Dihydro-5,7-dimethyl-6-oxo-7-phenyl-6H-indeno[4,5-d]-1,3-dioxol-2-carbonsäure vom Smp. 161-163°C und ihre Salze mit Basen herstellt.

8. Verfahren nach Anspruch 1, dadurch gekennzeichnet, dass man die 7,8-Dihydro-5,7-dimethyl-7-(2-fluorphenyl)-6-oxo-6H-indeno[4,5-d]-1,3-dioxo-2-carbonsäure und ihre Salze mit Basen herstellt.

9. Verfahren nach Anspruch 1, dadurch gekennzeichnet, dass man die 6,7-Dihydro-6-methyl-5-oxo-6-phenyl-5H-indeno[4,5-d]-1,4-dioxol-2-carbonsäure und ihre Salze mit Basen herstellt.

10. Verfahren nach Anspruch 1, dadurch gekennzeichnet, dass man die 6,7-Dihydro-6-(4-fluorphenyl)-6-methyl-5-oxo-5H-indeno[4,5-d]-1,3-dioxol-2-carbonsäure und ihre Salze mit Basen herstellt.

11. Verfahren nach Anspruch 1, dadurch gekennzeichnet, dass man die 7,8-Dihydro-5,7-dimethyl-7-(4-fluorphenyl)-6-oxo-6H-indeno[4,5-d]-1,3-dioxol-2-carbonsäure, deren Natriumsalz-Monohydrat bei 188 bis 190°C schmilzt, und ihre Salze mit Basen herstellt.

12. Verfahren nach Anspruch 1, dadurch gekennzeichnet, dass man das Racemat des 7,8-Dihydro-5,7-dimethyl-6-oxo-7-phenyl-6H-indeno[4,5-d]-1,3-dioxo-2-carbonsäure-äthylester vom Smp. 96 bis 97°C herstellt.

13. Verfahren nach Anspruch 1, dadurch gekennzeichnet, dass man das Racemat des 7,8-Dihydro-5,7-dimethyl-6-oxo-7-phenyl-5H-indeno[4,5-d]-1,3-dioxol-2-carbonsäure-äthylester vom Smp. 93 bis 94°C herstellt.

**Claims for the Contracting States:**
BE, CH, DE, FR, GB, IT, LI, LU, NL, SE

1. A dioxaheterocyclic compound of the general formula I

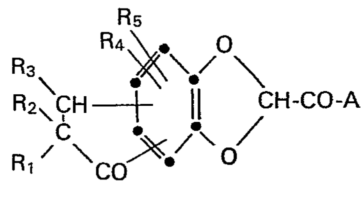

(I)

wherein $R_1$ is phenyl or pyridyl, each of which is unsubstituted or substituted by lower alkyl, lower alkoxy or halogen, $R_2$ is primary loweralkyl, each of $R_3$ and $R_4$ is, independently of the other, hydrogen or lower alkyl, $R_5$ is hydrogen, and A is the radical

-O-$R_6$, in which $R_6$ is hydrogen or an alkyl radical containing not more than 12 carbon atoms which is unsubstituted or substituted by lower alkoxy or halogen, or is 2- or 3-lower alkenyl, 2-lower alkynyl, or is phenyl-lower alkyl or cynnamyl in which the phenyl radical may be substituted in the same manner as indicated for $R_1$, or A is the radical $-N\langle^{R_7}_{R_8}$, in which each of $R_7$ and $R_8$, independently of the other, is hydrogen or lower alkyl, or, $R_7$ and $R_8$ are bonded to one another and, together with the adjacent nitrogen atom, are unsubstituted or lower alkyl-substituted tetra- to hexamethyleneimino or 4-morpholinyl, and the divalent radical in formula I is either in the 4,5- or the 5,6-position and correspondingly $R_4$ and $R_5$ are in positions 6 and 7, or 4 and 7, respectively, of the benzodioxole skeleton, in the form of a mixture of racemates, a racemate, a mixture of diastereoisomeric antipodes or an optical antipode, or a salt of a compound of the general formula I, wherein A is $OR_6$, in which $R_6$ is hydrogen, with a base, with the radicals qualified by the term «lower» containing not more than 7 carbon atoms.

2. A compound of the general formula I according to claim 1, wherein $R_1$ is phenyl or pyridyl, each of which is unsubstituted or substituted by lower alkyl, lower alkoxy or halogen, $R_2$ is primary lower alkyl, $R_3$ is hydrogen or lower alkyl, $R_5$ is hydrogen and A is $OR_6$, in which $R_6$ is hydrogen or lower alkyl, and $R_4$ is lower alkyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

3. A compound of the general formula I according to claim 1, wherein $R_1$ is phenyl which is unsubstituted or substituted by lower alkyl, lower alkoxy or halogen, $R_2$ is primary lower alkyl, each of $R_3$ and $R_5$ is hydrogen and A is $OR_6$, in which $R_6$ is hydrogen or lower alkyl, and $R_4$ is lower alkyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

4. A compound of the general formula I according to claim 1, wherein $R_1$ is phenyl which is unsubstituted or substituted by lower alkyl, lower alkoxy or halogen, $R_2$ is primary lower alkyl, each of $R_3$ and $R_5$ is hydrogen and A is $OR_6$, in which $R_6$ is hydrogen or lower alkyl, and $R_4$ is lower alkyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

5. A compound of the general formula I according to claim 1, wherein $R_1$ is phenyl which is unsubstituted or substituted by lower alkyl, lower alkoxy or fluorine, $R_2$ is methyl, each of $R_3$ and $R_5$ is hydrogen and A is $OR_6$, in which $R_6$ is hydrogen or lower alkyl, and $R_4$ is methyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

6. 7,8-Dihydro-5,7-dimethyl-6-oxo-7-phenyl-6H-indeno[4,5-d]-1,3-dioxole-2-carboxylic acid in the form of a mixture of racemates, a racemate, a mixture of diastereoisomeric antipodes or an optical antipode, or a salt thereof with a base.

7. The racemate of 7,8-dihydro-5,7-dimethyl-6-oxo-7-phenyl-6H-indeno[4,5-d]-1,3-dioxole-2-carboxylic acid having a melting point of 161-163°C, or a salt thereof with a base.

8. 7,8-Dihydro-5,7-dimethyl-7-(2-fluorophenyl)-6-oxo-6H-indeno[4,5-d]-1,3-dioxole-2-carboxylic acid, or a salt thereof with a base.

9. 6,7-Dihydro-6-methyl-5-oxo-6-phenyl-5H-indeno[4,5-d]-1,4-dioxole-2-carboxylic acid, or a salt thereof with a base.

10. 6,7-Dihydro-6-(4-fluorophenyl)-6-methyl-5-oxo-5H-indeno[4,5-d]-1,3-dioxole-2-carboxylic acid, or a salt thereof with a base.

11. 7,8-Dihydro-5,7-dimethyl-7-(4-fluorophenyl)-6-oxo-6H-indeno[4,5-d]-1,3-dioxole-2-carboxylic acid, the sodium salt monohydrate of which melts at 188-190°C, or a salt thereof with a base.

12. The racemate of ethyl 7,8-dihydro-5,7-dimethyl-6-oxo-7-phenyl-6H-indeno[4,5-d]-1,3-dioxole-2-carboxylate having a melting point of 96-97°C.

13. The racemate of ethyl 7,8-dihydro-5,7-dimethyl-6-oxo-7-phenyl-6H-indeno[4,5-d]-1,3-dioxole-2-carboxylate having a melting point of 93-94°C.

14. A pharmaceutically composition containing a compound according to any one of claims 1 to 13 or a pharmaceutically acceptable salt of a compound capable of salt-formation according to any one of claims 1 to 11.

15. A compound according to any of claims 1 to 13 or a pharmaceutically acceptable salt of a compound capable of salt-formation according to any one of claims 1 to 11 for use in a prophylactic and/or therapeutic method of treating the animal or human body.

16. Use of a compound according to any one of claims 1 to 13 or a pharmaceutically acceptable salt of a compound capable of salt-formation according to any one of claims 1 to 11 as diuretic with additional uricosuric action.

17. A process for the preparation of a pharmaceutical composition, which process comprises mixing a compound according to any one of claims 1 to 13 or a pharmaceutically acceptable salt of a compound capable of salt-formation according to any of claims 1 to 11 with conventional adjuvants.

18. A process for the preparation of a dioxaheterocyclic compound of the general formula I according to claim 1, in which formula $R_1$, $R_2$, $R_3$, $R_4$, $R_5$ and A are as defined in claim 1, or of a salt thereof, which process comprises

a) reacting a compound of the general formula II

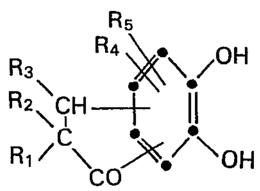

(II)

wherein $R_1$, $R_2$, $R_3$, $R_4$ and $R_5$ are as defined in claim 1, or a salt thereof, with a compound of the general formula III

Hal
>CH-CO-A (III)
Hal

wherein Hal is halogen and A is as defined in claim 1, or with a salt of such a compound, wherein A is $OR_6$, in which $R_6$ is hydrogen, or

b) in a compound of the general formula

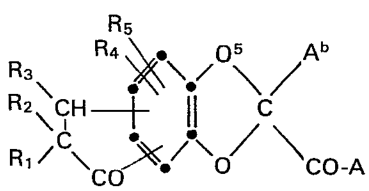

(IV)

wherein $A^b$ is carboxy, lower alkoxycarbonyl or acetyl and $R_1$, $R_2$, $R_3$, $R_4$, $R_5$ and A are as defined in claim 1, replacing the radical $A^b$ by hydrogen, or

c) to prepare a compound of the general formula I, wherein A is $OR_6$, in which $R_6$ is hydrogen, and $R_1$, $R_2$, $R_3$, $R_4$ and $R_5$ are as defined in claim 1, or a salt of this compound, in a compound of the general formula V

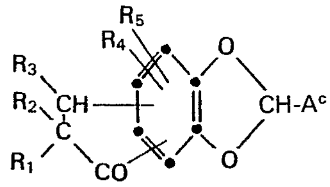

(V)

wherein $A^c$ is a group that can be converted into the carboxyl group and $R_1$, $R_2$, $R_3$, $R_4$ and $R_5$ are as defined in claim 1, converting the group $A^c$ into the carboxyl group in free or salt form, or

d) to prepare a compound of the general formula I, wherein A is as defined in claim 1, with the exception of a radical $OR_6$, in which $R_6$ is hydrogen, and $R_1$, $R_2$, $R_3$, $R_4$ and $R_5$ are as defined in claim 1, in a compound of the general formula VI

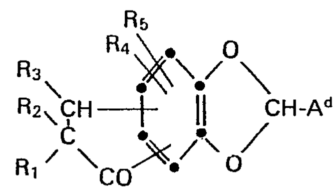

(VI)

wherein $A^d$ is a radical that can be converted into a radical $-CO-A_1$, in which $A_1$ has the same meaning as A as defined in claim 1, with the exception of a radical $OR_6$, in which $R_6$ is hydrogen, and $R_1$, $R_2$, $R_3$, $R_4$ and $R_5$ are as defined in claim 1, converting the radical $A^d$ into the radical $-CO-A_1$,

and, if desired, converting a resultant compound of the general formula I in a manner known per se into another compound of the general formula I, and/or separating a compound of the general formula I obtained in the form of a mixture of racemates into the individual racemates, and/or resolving a compound of the general formula I obtained in the form of a racemate or a mixture of diastereoisomeric antipodes into the optical antipodes, and/or converting a resultant compound of the general formula I, wherein A is $OR_6$, in which $R_6$ is hydrogen, into a salt with a base, or setting such a compound free from a resultant salt, or converting a resultant compound of the general formula I of basic character into an acid addition salt, or setting such a compound free from a resultant salt.

**Claims for the Contracting State:** AT

1. A process for the preparation of a dioxaheterocyclic compound of the general formula I

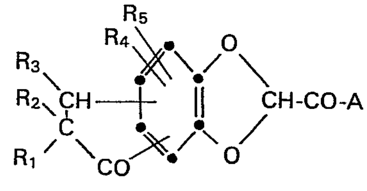

(I)

wherein $R_1$ is phenyl or pyridyl, each of which is unsubstituted or substituted by lower alkyl, lower alkoxy or halogen, $R_2$ is primary lower alkyl, each of $R_3$ and $R_4$ is, independently of the other, hydrogen or lower alkyl, $R_5$ is hydrogen, and A is the radical $-O-R_6$, in which $R_6$ is hydrogen or an alkyl radical containing not more than 12 carbon atoms which is unsubstituted or substituted by lower alkoxy or halogen, or is 2- or 3-lower alkenyl, 2-lower alkynyl, or is phenyl-lower alkyl or cynnamyl in which the phenyl radical may be substituted in the same manner as indicated for $R_1$, or A is the radical $-N\!<^{R_7}_{R_8}$, in which each of $R_7$ and $R_8$, independently of the other, is hydrogen or lower alkyl, or, $R_7$ and $R_8$ are bonded to one another and, together with the adjacent nitrogen atom, are unsubstituted or lower alkyl-substituted tetra- to hexamethyleneimino or 4-morpholinyl, and the divalent radical in formula I is either in the 4,5- or the 5,6-position and correspondingly $R_4$ and $R_5$ are in positions 6 and 7, or 4 and 7, respectively, of the benzodioxole skeleton, in the form of a mixture of racemates, a racemate, a mixture of diastereoisomeric antipodes or an optical antipode, or a salt of a compound of the general formula I, wherein A is $OR_6$, in which $R_6$ is hydrogen, with a base, with the radicals qualified by the term «lower» containing not more than 7 carbon atoms, which process comprises

a) reacting a compound of the general formula II

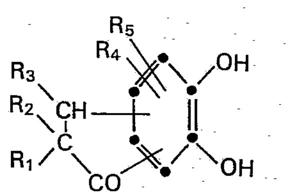

(II)

wherein $R_1$, $R_2$, $R_3$, $R_4$ and $R_5$ are as defined in claim 1, or a salt thereof, with a compound of the general formula III

$$\begin{matrix} Hal \\ & >CH-CO-A \\ Hal \end{matrix} \qquad (III)$$

wherein Hal is halogen and A is as defined in claim 1, or with a salt of such a compound, wherein A is $OR_6$, in which $R_6$ is hydrogen, or

b) in a compound of the general formula

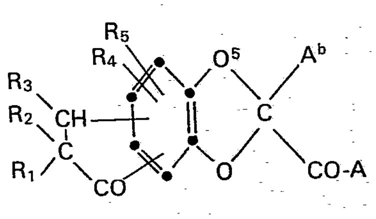

(IV)

wherein $A^b$ is carboxy, lower alkoxycarbonyl or acetyl and $R_1$, $R_2$, $R_3$, $R_4$, $R_5$ and A are as defined in claim 1, replacing the radical $A^b$ by hydrogen, or

c) to prepare a compound of the general formula I, wherein A is $OR_6$, in which $R_6$ is hydrogen, and $R_1$, $R_2$, $R_3$, $R_4$ and $R_5$ are as defined in claim 1, or a salt of this compound, in a compound of the general formula V

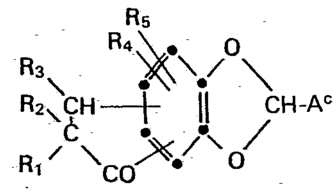

(V)

wherein $A^c$ is a group that can be converted into the carboxyl group and $R_1$, $R_2$, $R_3$, $R_4$ and $R_5$ are as defined in claim 1, converting the group $A^c$ into the carboxyl group in free or salt form, or

d) to prepare a compound of the general formula I, wherein A is as defined in claim 1, with the exception of a radical $OR_6$, in which $R_6$ is hydrogen, and $R_1$, $R_2$, $R_3$, $R_4$ and $R_5$ are as defined in claim 1, in a compound of the general formula VI

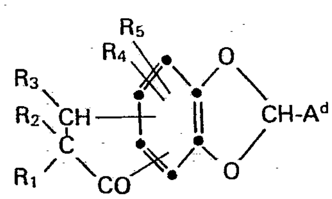

(VI)

wherein $A^d$ is a radical that can be converted into a radical $-CO-A_1$, in which $A_1$ has the same meaning as A as defined in claim 1, with the exception of a radical $OR_6$, in which $R_6$ is hydrogen, and $R_1$, $R_2$, $R_3$, $R_4$ and $R_5$ are as defined in claim 1, converting the radical $A^d$ into the radical $-CO-A_1$,

and, if desired, converting a resultant compound of the general formula I in a manner known per se into another compound of the general formula I, and/or separating a compound of the general formula I obtained in the form of a mixture of racemates into the individual racemates, and/or resolving a compound of the general formula I obtained in the form of a racemate or a mixture of diastereoisomeric antipodes into the optical antipodes, and/or converting a resultant compound of the general formula I, wherein A is $OR_6$, in which $R_6$ is hydrogen, into a salt with a base, or setting such a compound free from a resultant salt, or converting a resultant compound of the general formula I of basic character into an acid addition salt, or setting such a compound free from a resultant salt.

2. A process according to claim 1, which comprises preparing a compound of the general formula I according to claim 1, wherein $R_1$ is phenyl or pyridyl, each of which is unsubstituted or substituted by

lower alkyl, lower alkoxy or halogen, $R_2$ is primary lower alkyl, $R_3$ is hydrogen or lower alkyl, $R_5$ is hydrogen and A is $OR_6$, in which $R_6$ is hydrogen or lower alkyl, and $R_4$ is lower alkyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

3. A process according to claim 1, which comprises preparing a compound of the general formula I according to claim 1, wherein $R_1$ is phenyl which is unsubstituted or substituted by lower alkyl, lower alkoxy or halogen, $R_2$ is primary lower alkyl, each of $R_3$ and $R_5$ is hydrogen and A is $OR_6$, in which $R_6$ is hydrogen or lower alkyl, and $R_4$ is lower alkyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

4. A process according to claim 1, which comprises preparing a compound of the general formula I according to claim 1, wherein $R_1$ is phenyl which is unsubstituted or substituted by lower alkyl, lower alkoxy or halogen, $R_2$ is primary lower alkyl, each of $R_3$ and $R_5$ is hydrogen and A is $OR_6$, in which $R_6$ is hydrogen or lower alkyl, and $R_4$ is methyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

5. A process according to claim 1, which comprises preparing a compound of the general formula I according to claim 1, wherein $R_1$ is phenyl which is unsubstituted or substituted by lower alkyl, lower alkoxy or fluorine, $R_2$ is methyl, each of $R_3$ and $R_5$ is hydrogen and A is $OR_6$, in which $R_6$ is hydrogen or lower alkyl, and $R_4$ is methyl bonded in the 5-position of the tricyclic compound and at the same time the divalent radical in formula I is in the 4,5-position of the benzodioxole skeleton, or $R_4$ is hydrogen and at the same time the divalent radical in formula I is in the 5,6-position of the benzodioxole skeleton, in the form of a mixture of racemates, a racemate, a mixture of diastereoisomeric antipodes or an optical antipode, or a pharmaceutically acceptable salt of such a compound, wherein A is $OR_6$, in which $R_6$ is hydrogen, with a base, with the radicals qualified by the term «lower» containing not more than 7 carbon atoms.

6. A process according to claim 1, which comprises preparing 7,8-dihydro-5,7-dimethyl-6-oxo-7-phenyl-6H-indeno[4,5-d]-1,3-dioxole-2-carboxylic acid in the form of a mixture of racemates, a racemate, a mixture of diastereoisomeric antipodes or an optical antipode, or a salt thereof with a base.

7. A process according to claim 1, which comprises preparing 7,8-dihydro-5,7-dimethyl-6-oxo-7-phenyl-6H-indeno[4,5-d]-1,3-dioxole-2-carboxylic acid having a melting point of 161-163°C, or a salt thereof with a base.

8. A process according to claim 1, which comprises preparing 7,8-dihydro-5,7-dimethyl-7-(2-fluorophenyl)-6-oxo-6H-indeno[4,5-d]-1,3-dioxole-2-carboxylic acid, or a salt thereof with a base.

9. A process according to claim 1, which comprises preparing 6,7-dihydro-6-methyl-5-oxo-6-phenyl-5H-indeno[4,5-d]-1,4-dioxole-2-carboxylic acid, or a salt thereof with a base.

10. A process according to claim 1, which comprises preparing 6,7-dihydro-6-(4-fluorophenyl)-6-methyl-5-oxo-5H-indeno[4,5-d]-1,3-dioxole-2-carboxylic acid, or a salt thereof with a base.

11. A process according to claim 1, which comprises preparing 7,8-Dihydro-5,7-dimethyl-7-(4-fluorophenyl)-6-oxo-6H-indeno[4,5-d]-1,3-dioxole-2-carboxylic acid, the sodium salt monohydrate of which melts at 188-190°C, or a salt thereof with a base.

12. A process according to claim 1, which comprises preparing the racemate of ethyl 7,8-dihydro-5,7-dimethyl-6-oxo-7-phenyl-6H-indeno[4,5-d]-1,3-dioxole-2-carboxylate having a melting point of 96-97°C.

13. A process according to claim 1, which comprises preparing the racemate of ethyl 7,8-dihydro-5,7-dimethyl-6-oxo-7-phenyl-6H-indeno[4,5-d]-1,3-dioxole-2-carboxylate having a melting point of 93-94°C.

**Revendications pour les Etats contractants:**
BE, CH, DE, FR, GB, IT, LI, LU, NL, SE

1. Composés dioxahétérocycliques de formule générale I

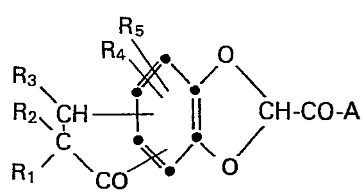

(I)

dans laquelle $R_1$ représente un groupe phényle ou pyridyle non substitué ou substitué par des groupes alkyle inférieurs, alcoxy inférieurs ou des halogènes, $R_2$ représente un groupe alkyle inférieur primaire, $R_3$ et $R_4$ représentent, indépendamment l'un de l'autre, l'hydrogène ou un groupe alkyle inférieur, $R_5$ représente l'hydrogène et A le reste -O-$R_6$ dans lequel $R_6$ représente l'hydrogène ou un groupe alkyle à 12 atomes de carbone au maximum, non substitué ou substitué par des groupes alcoxy inférieurs ou des halogènes, un groupe 2- ou 3-alcényle inférieur, 2-alcynyle inférieur, phényl-alkyle inférieur ou cinnamyle dans lesquels le reste phényle peut être substitué comme indiqué pour $R_1$, ou le reste $-N\begin{matrix} R_7 \\ R_8 \end{matrix}$, dans lequel $R_7$ et $R_8$ représentent, indépendamment l'un de l'autre, l'hydrogène ou un groupe alkyle inférieur, ou bien forment ensemble et avec l'atome d'azote voisin un groupe tétra-bis-hexaméthylène-imino ou 4-morpholinyle éventuellement substitué par des groupes alkyle inférieurs, et le reste divalent en question se trouve soit en position 4,5 soit en position 5,6 et, en correspondance, $R_4$ et $R_5$ se trouvent dans les positions respectives 6 et 7 ou 4 et 7 de la partie benzodioxole de la molécule, à l'état de mélanges de racémates, de racémates, de mélanges diastéréoisomères d'antipodes ou d'antipodes optiques, ainsi que les sels des composés de formule générale I dans lesquels A = $OR_6$ et $R_6$ représente l'hydrogène, et de bases, les reste appelés «inférieurs» contenant au maximum 7 atomes de carbone.

2. Composés de formule générale I de la revendication 1, dans lesquels $R_1$ représente un groupe phényle ou pyridyle non substitué ou substitué par des groupes alkyle inférieurs, alcoxy inférieurs ou des halogènes, $R_2$ représente un groupe alkyle inférieur primaire, $R_3$ représente l'hydrogène ou un groupe alkyle inférieur, $R_5$ représente l'hydrogène et A = $OR_6$ dans lequel $R_6$ représente l'hydrogène ou un groupe alkyle inférieur, et $R_4$ représente un groupe alkyle inférieur relié en position 5 du noyau tricyclique, le reste divalent en question se trouvant alors en position 4,5 de la partie benzodioxole de la molécule, ou bien $R_4$ représente l'hydrogène et le reste divalent en question est alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés pour lesquels A = $OR_6$ et $R_6$ représente l'hydrogène, et de bases, les restes appelés «inférieurs» contenant au maximum 7 atomes de carbone.

3. Composés de formule générale I de la revendication 1, dans lesquels $R_1$ représente un groupe phényle non substitué ou substitué par des groupes alkyle inférieurs, alcoxy inférieurs ou des halogènes, $R_2$ représente un groupe alkyle inférieur primaire, $R_3$ et $R_5$ représentent chacun l'hydrogène et A = $OR_6$ dans lequel $R_6$ représente l'hydrogène ou un groupe alkyle inférieur, et $R_4$ représente un groupe alkyle inférieur relié en position 5 du noyau tricyclique, le reste divalent en question se trouvant alors en position 4,5 de la partie benzodioxole de la molécule, ou bien $R_4$ représente l'hydrogène et le reste divalent en question est alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés pour lesquels A = $OR_6$ et $R_6$ représente l'hydrogène, et de bases, les restes appelés «inférieurs» contenant au maximum 7 atomes de carbone.

4. Composés de formule générale I de la revendication 1, dans lesquels $R_1$ représente un groupe phényle non substitué ou substitué par des groupes alkyle inférieurs alcoxy inférieurs ou des halogènes, $R_2$ représente un groupe alkyle inférieur primaire, $R_3$ et $R_5$ représentent chacun l'hydrogène et A = $OR_6$ dans lequel $R_6$ représente l'hydrogène ou un groupe alkyle inférieur, et $R_4$ est un groupe méthyle fixé en position 5 du noyau tricyclique, le reste divalent en question étant alors en position 4,5 de la partie benzodioxole de la molécule, ou bien $R_4$ représente l'hydrogène et le reste divalent en question est alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés pour lesquels A représente $OR_6$ et $R_6$ l'hydrogène, et de bases, les restes appelés «inférieurs» contenant au maximum 7 atomes de carbone.

5. Composés de formule générale I de la revendication 1, dans lesquels $R_1$ représente un groupe phényle non substitué ou substitué par des groupes alkyle inférieurs, alcoxy inférieurs ou le fluor, $R_2$ représente un groupe méthyle, $R_3$ et $R_5$ représentent chacun l'hydrogène et A représente $OR_6$ dans lequel $R_6$ représente l'hydrogène ou un groupe alkyle inférieur, et $R_4$ représente un groupe méthyle fixé en position 5 du noyau tricyclique, le reste divalent en question étant alors en position 4,5 de la partie benzodioxole de la molécule, ou bien $R_4$ représente l'hydrogène et le reste divalent en question se trouve alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés dans lesquels A représente $OR_6$ et $R_6$ l'hydrogène, et de bases, les restes appelés «inférieurs» contenant au maximum 7 atomes de carbone.

6. L'acide 7,8-dihydro-5,7-diméthyl-6-oxo-7-phényl-6H-indéno[4,5-d]-1,3-dioxole-2-carboxylique à l'état de mélange de racémates, de racémate, de mélange diastéréoisomères d'antipodes ou d'antipodes optiques, et ses sels de bases.

7. Le racémate de l'acide 7,8-dihydro-5,7-diméthyl-6-oxo-7-phényl-6H-indéno[4,5-d]-1,3-dioxole-2-carboxylique fondant à 161-163°C et ses sels de bases.

8. L'acide 7,8-dihydro-5,7-diméthyl-7-(2-fluorophényl)-6-oxo-6H-indéno[4,5-d]-1,3-dioxo-2-carboxylique et ses sels de bases.

9. L'acide 6,7-dihydro-6-méthyl-5-oxo-6-phenyl-5H-indéno[4,5-d]-1,4-dioxole-2-carboxylique et ses sels de bases.

10. L'acide 6,7-dihydro-6-(4-fluorophényl)-6-

-méthyl-5-oxo--5H-indéno[4,5-d]-1,3-dioxole-2--carboxylique et ses sels de bases.

11. L'acide 7,8-dihydro-5,7-diméthyl-7-(4-fluorophényl)-6-oxo-6H-indéno[4,5-d]-1,3-dioxole-2--carboxylique, dont le sels de sodium monohydraté fond à 188-190°C, et ses sels de bases.

12. Le racémate du 7,8-dihydro-5,7-diméthyl-6--oxo-7-phényl-6H-indéno[4,5-d]-1,3-dioxo-2-carboxylate d'éthyle fondant à 96-97°C.

13. Le racémate du 7,8-dihydro-5,7-diméthyl-6--oxo-7-phényl-6H-indéno[4,5-d]-1,3-dioxole-2-carboxylate d'éthyle fondant à 93-94°C.

14. Compositions pharmaceutiques contenant un composé selon l'une des revendications 1 à 13 ou un sel acceptable pour l'usage pharmaceutique d'un composé selon l'une des revendications 1 à 11 apte à la salification.

15. Composés selon l'une des revendications 1 à 13, ou sels acceptables pour l'usage pharmaceutique de composés selon l'une des revendications 1 à 11 aptes à la salification, pour l'utilisation dans un procédé pour le traitement prophylactique et/ou thérapeutique de l'organisme humain ou animal.

16. Composés selon l'une des revendications 1 à 13 et sels acceptables pour l'usage pharmaceutique de composés selon l'une des revendications 1 à 11 aptes à la salification, pour l'utilisation en tant que diurétiques avec activité additionnelle uricosurique.

17. Procédé de préparation de médicaments, caractérisé en ce que l'on mélange des composés selon l'une des revendications 1 à 13 ou des sels acceptables pour l'usage pharmaceutique de composés selon l'une des revendications 1 à 11 aptes à la salification, avec des additifs usuels.

18. Procédé de préparation des composés dioxahétérocycliques de formule générale I de la revendication 1, dans laquelle $R_1$, $R_2$, $R_3$, $R_4$, $R_5$ et A ont les significations indiquées en référence à cette formule, et des sels de ces composés, caractérisé en ce que:

a) on fait réagir un composé de formule générale II

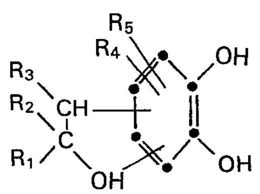

(II)

dans laquelle $R_1$, $R_2$, $R_3$, $R_4$ et $R_5$ ont les significations indiquées dans la revendication 1, ou un sel d'un tel composé, avec un composé de formule générale III

$$\begin{matrix} Hal \\ & >CH\text{-}CO\text{-}A \\ Hal \end{matrix} \qquad (III)$$

dans laquelle Hal représente un halogéne et A a la signification indiquée dans la revendication 1, ou un sel d'un tel composé dans lequel $A = OR_6$ et $R_6$ représente l'hydrogène, ou bien

b) dans un composé de formule générale

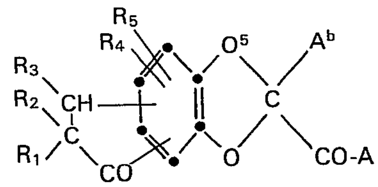

(IV)

dans laquelle $A^b$ représente un groupe carboxy, (alcoxy inférieur)-carbonyle ou acétyle et $R_1$, $R_2$, $R_3$, $R_4$, $R_5$ et A ont les significations indiquées dans la revendication 1, on remplace le reste $A^b$ par l'hydrogène, ou bien

c) pour préparer un composé de formule générale I dans laquelle A = $OR_6$ et $R_6$ représente l'hydrogène, $R_1$, $R_2$, $R_3$, $R_4$ et $R_5$ ayant les significations indiquées dans la revendication 1, ou un sel de ce composé, on part d'un composé de formula générale V

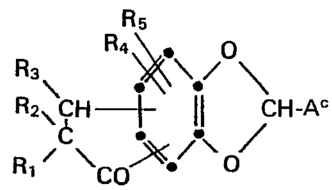

(V)

dans laquelle $A^c$ représente un groupe convertible en le groupe carboxy et $R_1$, $R_2$, $R_3$, $R_4$ et $R_5$ ont les significations indiquées dans la revendication 1, et on convertit le groupe $A^c$ en le groupe carboxy à l'état libre ou à l'état de sel, ou

d) pour préparer un composé de formule générale I dans laquelle A a les significations indiquées dans la revendication 1 à l'exception d'un reste $OR_6$ dans lequel $R_6$ représente l'hydrogène et $R_1$, $R_2$, $R_3$, $R_4$ et $R_5$ ont les significations indiquées dans la revendication 1, on part d'un composé de formule générale VI

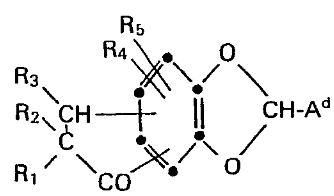

(VI)

dans laquelle $A^d$ représente un reste convertible en un reste $-CO-A_1$ dans lequel $A_1$ a les significations indiquées pour A dans la revendication 1 à l'exception d'un reste $OR_6$ dans lequel $R_6$ représente l'hydrogène, $R_1$, $R_2$, $R_3$, $R_4$ et $R_5$ ayant les significations indiquées dans la revendication 1, et on convertit le reste $A^d$ en le reste $-CO-A_1$, et si on le désire, on convertit un composé obtenu répondant à la formule générale I, de manière connue en soi, en un autre composé de formule générale I, et/ou on sépare un composé de formule générale I obtenu à l'état de mélange de racémates en les racémates et/ou on résout un

composé de formule générale I obtenu à l'état de racémate ou de mélange diastéréoisomère d'antipodes en les antipodes optiques, et/ou on convertit un composé obtenu, répondant à la formule générale I dans laquelle A = $OR_6$ et $R_6$ représente l'hydrogène, en un sel à l'aide d'une base, ou bien on libère un tel composé à partir d'un sel obtenu, ou bien on convertit un composé obtenu répondant à la formule générale I et ayant un caractère basique en un sel par addition avec un acide ou on libère un tel composé à partir d'un sel obtenu.

**Revendications pour l'Etat contractant:** AT

1. Procédé de préparation de composés dioxahétérocycliques de formule générale I

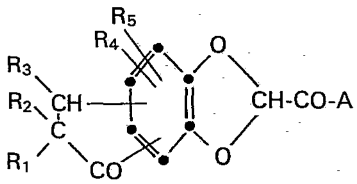

(I)

dans laquelle $R_1$ représente un groupe phényle ou pyridyle non substitué ou substitué par des groupes alkyle inférieurs, alcoxy inférieurs ou des halogènes, $R_2$ représente un groupe alkyle inférieur primaire, $R_3$ et $R_4$ représentent, indépendamment l'un de l'autre, l'hydrogène ou un groupe alkyle inférieur, $R_5$ représente l'hydrogène et A le reste $-O-R_6$ dans lequel $R_6$ représente l'hydrogène ou un groupe alkyle à 12 atomes de carbone au maximum, non substitué ou substitué par des groupes alcoxy inférieurs ou des halogènes, un groupe 2- ou 3-alcényle inférieur, 2-alcynyle inférieur, phényl-(alkyle inférieur) ou cinnamyle dans lesquels le reste phényle peut être substitué comme indiqué pour $R_1$, ou bien le reste $-N<^{R_7}_{R_8}$, dans lequel $R_7$ et $R_8$ représentent, indépendamment l'un de l'autre, l'hydrogène ou un groupe alkyle inférieur, ou bien forment ensemble et avec l'atome d'azote voisin un groupe tétra-bis-hexaméthylène-imino ou 4-morpholinyle éventuellement substitué par des groupes alkyle inférieurs, et le reste divalent en question se trouve soit en position 4,5 soit en position 5,6, $R_4$ et $R_5$ se trouvant alors en correspondance dans les positions respectives 6 et 7 ou 4 et 7 de la partie benzodioxole de la molécule, et de leurs sels, les restes appelés «inférieurs» contenant au maximum 7 atomes de carbone, caractérisé en ce que:

a) on fait réagir un composé de formule générale II

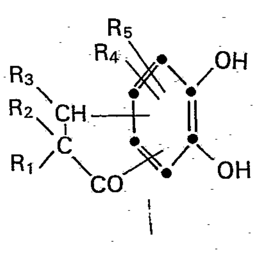

(II)

dans laquelle $R_1$, $R_2$, $R_3$, $R_4$ et $R_5$ ont les significations indiquées en référence à la formule I, ou un sel d'un tel composé, avec un composé de formule générale III

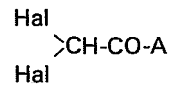

(III)

dans laquelle Hal représente un halogène et A a la signification indiquée en référence à la formule I, ou un sel d'un tel composé dans lequel A = $OR_6$ et $R_6$ représente l'hydrogène, ou bien

b) dans un composé de formule générale

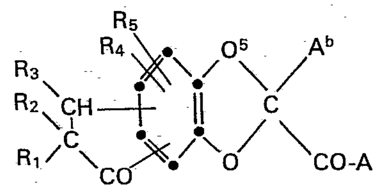

(IV)

dans laquelle $A^b$ représente un groupe carboxy, (alcoxy inférieur)-carbonyle ou acétyle et $R_1$, $R_2$, $R_3$, $R_4$ et $R_5$ et A ont les significations indiquées en référence à la formule I, on remplace $A^b$ par l'hydrogène, ou bien

c) pour préparer un composé de formule générale I dans laquelle A = $OR_6$ et $R_6$ représente l'hydrogène, $R_1$, $R_2$, $R_3$, $R_4$ et $R_5$ ayant les significations indiquées en référence à la formule I, ou un sel de ce composé, on part d'un composé de formule générale V

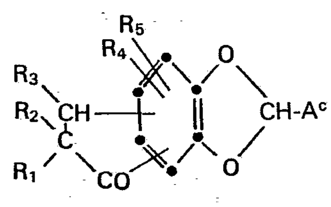

(V)

dans laquelle $A^c$ représente un groupe convertible en le groupe carboxy et $R_1$, $R_2$, $R_3$, $R_4$ et $R_5$ ont les significations indiquées en référence à la formule I, et on convertit le groupe $A^c$ en le groupe carboxy à l'état libre ou à l'état de sel, ou

d) pour préparer un composé de formule générale I dans laquelle A a les significations indiquées en référence à la formule I, à l'exception d'un reste $OR_6$ dans lequel $R_6$ représente l'hydrogène et $R_1$, $R_2$, $R_3$, $R_4$ et $R_5$ ont les significations indiquées en référence à la formule I, on part d'un composé de formule générale VI

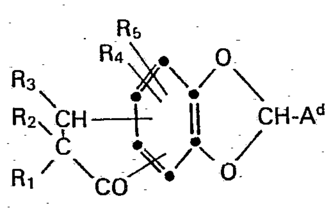

(VI)

dans laquelle

$A^d$ représente un reste convertible en un reste -CO-$A_1$ dans lequel $A_1$ a les significations indiquées pour A en référence à la formule I, à l'exception d'un reste $OR_6$ dans lequel $R_6$ représente l'hydrogène, $R_1$, $R_2$, $R_3$, $R_4$ et $R_5$ ayant les significations indiquées en référence à la formule I, et on convertit le reste $A^d$ en le reste -CO-$A_1$, et si on le désire, on convertit un composé obtenu répondant à la formule générale I, de manière connue en soi, en un autre composé de formule générale I, et/ou on sépare un composé de formule générale I obtenu à l'état de mélange de racémates en les racémates et/ou on résout un composé de formule générale I obtenu à l'état de racémate ou de mélange diastéréoisomère d'antipodes en les antipodes optiques, et/ou on convertit un composé obtenu, répondant à la formule générale I dans laquelle $A = OR_6$ et $R_6$ représente l'hydrogène, en un sel à l'aide d'une base, ou bien ou libère un tel composé à partir d'un sel obtenu, ou bien on convertit un composé obtenu répondant à la formule générale I et ayant un caractère basique en un sel par addition avec un acide ou on libère un tel composé à partir d'un sel obtenu.

2. Procédé selon la revendication 1, caractérisé en ce que l'on prépare des composés de formule générale I de la revendication 1, dans lesquels $R_1$ représente un groupe phényle ou pyridyle non substitué ou substitué par des groupes alkyle inférieurs, alcoxy inférieurs ou des halogènes, $R_2$ représente un groupe alkyle inférieur primaire, $R_3$ représente l'hydrogène ou un groupe alkyle inférieur, $R_5$ représente l'hydrogène et $A = OR_6$ dans lequel $R_6$ représente l'hydrogène ou un groupe alkyle inférieur, et $R_4$ représente un groupe alkyle inférieur relié en position 5 du noyau tricyclique, le reste divalent en question se trouvant alors en position 4,5 de la partie benzodioxole de la molécule, ou bien $R_4$ représente l'hydrogène et le reste divalent en question est alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés pour lesquels $A = OR_6$ et $R_6$ représente l'hydrogène, et de bases, les restes appelés «inférieurs» contenant au maximum 7 atomes de carbone.

3. Procédé selon la revendication 1, caractérisé en ce que l'on prépare des composés de formule générale I de la revendication 1, dans lesquels $R_1$ représente un groupe phényle non substitué ou substitué par des groupes alkyle inférieurs, alcoxy inférieurs ou des halogènes, $R_2$ représente un groupe alkyle inférieur primaire, $R_3$ et $R_5$ représentent chacun l'hydrogène et $A = OR_6$ dans lequel $R_6$ représente l'hydrogène ou un groupe alkyle inférieur, et $R_4$ est un groupe alkyle inférieur relié en position 5 du noyau tricyclique, le reste divalent en question étant alors en position 4,5 de la partie benzodioxole de la molécule, ou bien $R_4$ représente l'hydrogène et le reste divalent en question est alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés pour lesquels $A = OR_6$ et $R_6$ représente l'hydrogène, et de bases, les restes appelés «inférieurs» contenant au maximum 7 atomes de carbone.

4. Procédé selon la revendication 1, caractérisé en ce que l'on prépare des composés de formule générale I de la revendication 1, dans lesquels $R_1$ représente un groupe phényle non substitué ou substitué par des groupes alkyle inférieurs, alcoxy inférieurs ou des halogènes, $R_2$ représente un groupe alkyle inférieur primaire, $R_3$ et $R_5$ représentent chacun l'hydrogène et $A = OR_6$ dans lequel $R_6$ représente l'hydrogène ou un groupe alkyle inférieur, et $R_4$ est un groupe méthyle fixé en position 5 du noyau tricyclique, le reste divalent en question étant alors en position 4,5 de la partie benzodioxole de la molécule, ou bien $R_4$ représente l'hydrogène et le reste divalent en question est alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés dans lesquels A représente $OR_6$ et $R_6$ l'hydrogène, et de bases, les restes appelés «inférieurs» contenant au maximum 7 atomes de carbone.

5. Procédé selon la revendication 1, caractérisé en ce que l'on prépare des composés de formule générale I de la revendication 1, dans lesquels $R_1$ représente un groupe phényle non substitué ou substitué par des groupes alkyle inférieurs, alcoxy inférieurs ou le fluor, $R_2$ représente un groupe méthyle, $R_3$ et $R_5$ représentent chacun l'hydrogène et A représente $OR_6$ dans lequel $R_6$ représente l'hydrogène ou un groupe alkyle inférieur, et $R_4$ représente un groupe méthyle fixé en position 5 du noyau tricyclique, le reste divalent en question étant alors en position 4,5 de la partie benzodioxole de la molécule, ou bien $R_4$ représente l'hydrogène et le reste divalent en question se trouve alors en position 5,6 de la partie benzodioxole de la molécule, à l'état de mélanges de racémates, de racémates, de mélanges diastéréoisomères d'antipodes ou d'antipodes optiques, ainsi que les sels acceptables pour l'usage pharmaceutique de ces composés dans lesquels A représente $OR_6$ et $R_6$ l'hydrogène, et de bases, les restes appelés «inférieurs» contenant au maximum 7 atomes de carbone.

6. Procédé selon la revendication 1, caractérisé en ce que l'on prépare l'acide 7,8-dihydro-5,7-diméthyl-6-oxo-7-phényl-6H-indéno[4,5-d]-1,3-dioxole-2-carboxylique à l'état de mélange de racémates, de racémate, de mélange diastéréoisomères d'antipodes ou d'antipodes optiques, et ses sels de bases.

7. Procédé selon la revendication 1, caractérisé en ce que l'on prépare le racémate de l'acide 7,8--dihydro-5,7-diméthyl-6-oxo-7-phényl-6H-indéno-[4,5-d]-1,3-dioxole-2-carboxylique fondant à 161-163°C et ses sels de bases.

8. Procédé selon la revendication 1, caractérisé en ce que l'on prépare l'acide 7,8-dihydro-5,7-diméthyl-7-(2-fluorophényl)-6-oxo-6H-indéno[4,5-d]--1,3-dioxo-2-carboxylique et ses sels de bases.

9. Procédé selon la revendication 1, caractérisé

en ce que l'on prépare l'acide 6,7-dihydro-6-méthyl-5-oxo-6-phenyl-5H-indéno[4,5-d]-1,4-dioxole-2-carboxylique et ses sels de bases.

10. Procédé selon la revendication 1, caractérisé en ce que l'on prépare l'acide 6,7-dihydro-6-(4-fluorophényl)-6-méthyl-5-oxo-5H-indéno[4,5-d)-1,3-dioxole-2-carboxylique et ses sels de bases.

11. Procédé selon la revendication 1, caractérisé en ce que l'on prépare l'acide 7,8-dihydro-5,7-(4-fluorophényl)-6-oxo-6H-indéno[4,5-d)-1,3-dioxole-2-carboxylique, dont le sel de sodium monohydraté fond à 188-190°C, et ses sels de bases.

12. Procédé selon la revendication 1, caractérisé en ce que l'on prépare le racémate du 7,8-dihydro-5,7-diméthyl-6-oxo-7-phényl-6H-indéno[4,5-d)-1,3-dioxo-2-carboxylate d'éthyle, fondant à 96-97°C.

13. Procédé selon la revendication 1, caractérisé en ce que l'on prépare le racémate du 7,8-dihydro-5,7-diméthyl-6-oxo-7-phényl-6H-indéno[4,5-d)-1,3-dioxole-2-carboxylate d'éthyle fondant à 93-94°C.